# EUROPEAN PATENT SPECIFICATION

(11) **EP 4 039 182 B1**
(45) Date of publication and mention of the grant of the patent: **13.09.2023**
(21) Application number: 20930666.1
(22) Date of filing: 09.12.2020
(51) Int. Cl.: A61B 5/021, A61B 5/00, A61B 5/0225

(54) **BLOOD PRESSURE MEASUREMENT METHOD AND APPARATUS, AND ELECTRONIC DEVICE**
VERFAHREN, VORRICHTUNG UND ELEKTRONISCHES GERÄT ZUR BLUTDRUCKMESSUNG
PROCÉDÉ ET APPAREIL DE MESURE DE LA PRESSION ARTÉRIELLE, ET DISPOSITIF ÉLECTRONIQUE

(43) Date of publication of application: 10.08.2022
(73) Proprietor: SHENZHEN GOODIX TECHNOLOGY CO., LTD., Shenzhen, Guangdong 518045 (CN)
(72) Inventor: LI, Huafei, Guangdong 518045 (CN)
(74) Representative: Herrero & Asociados, S.L.
(86) International application number: PCT/CN2020/135009
(87) International publication number: WO 2022/120656

(56) References cited:
- WO-A1-2016/193423
- WO-A1-2019/053276
- CN-A- 107 405 090
- CN-A- 108 236 460
- CN-A- 108 523 867
- CN-A- 109 793 507
- CN-A- 110 123 294
- CN-A- 110 974 197
- CN-A- 111 990 980
- US-A1- 2010 241 011
- US-A1- 2020 163 561

## Description

### TECHNICAL FIELD

The present application relates to the field of electronic technologies, and in particular, to a blood pressure detection method, an apparatus and an electronic device.

### BACKGROUND

The blood pressure is one of the important parameters to measure the human cardiovascular system. It has important significance in disease diagnosis, treatment processes and prognosis judgment. At present, the mature blood pressure detection method on the market is a cuff-type detection method based on an auscultation method or an oscillometry method. Among them, the auscultation method requires a professional operator to judge the blood pressure based on the sound of brachial artery blood flow, which is suitable for medical scenarios. The oscillometric method is to first inflate a cuff to block arterial blood flow, then detect a gas pressure in the cuff and extract the weak pulse wave during the process of exhausting, and detect and obtain a blood pressure value according to a change of a pulse wave with the pressure in the cuff.

With the development of living standards, sphygmomanometers adopting the above cuff-type detection method have gradually entered an increasing number of daily use scenarios. For hypertensive patients, accuracy of blood pressure measurement and portability of the sphygmomanometer are very important. Although blood pressure measurement adopting a sphygmomanometer with a cuff-type detection method is relatively accurate, it is not easy to carry, cannot meet the needs of around-the-clock blood pressure monitoring, and cannot meet the needs of hypertensive patients.

Therefore, there are great application prospects and market values to provide a blood pressure detection apparatus with accurate blood pressure detection results, capable and convenient to carry.

US2010/241011A1 discloses an apparatus and methods for adaptive and autonomous calibration of pulse transit time measurements to obtain arterial blood pressure using arterial pressure variation. The apparatus and methods give pulse transit time (PTT) devices an ability to self-calibrate. The methods apply a distributed model with lumped parameters, and may be implemented, for example, using pulse transit time measurements derived from a wearable photoplethysmograph (PPG) sensor architecture with an intervening pressurizing mechanism.

### SUMMARY

The invention is set out in the appended set of claims.

An embodiment of the present application provides a blood pressure detection method, an apparatus, and an electronic device, having a relatively accurate blood pressure detection result and are convenient to detect.

In the first aspect, a blood pressure detection method is provided, to apply to a blood pressure detection apparatus, including:
acquiring blood pressure calibration information, the blood pressure calibration information being information obtained by processing a first pressure acting on a user and a first photo plethysmography (PPG) signal when the first pressure acts on the user;
acquiring a second photo plethysmography (PPG) signal, and processing the second PPG signal to obtain the user's initial blood pressure;
calibrating the initial blood pressure according to the blood pressure calibration information, and using the blood pressure obtained by calibration as the blood pressure of the user.

In the technical solution of the embodiment of the present application, when the second PPG signal is detected, accuracy of the blood pressure measured based on the second PPG signal is low. At this time, there is no need to resort to an external device such as a sphygmomanometer for assistance, but to directly acquire the blood pressure calibration information determined by the first PPG signal and the first pressure directly according to the blood pressure detection apparatus per se, the blood pressure calibration information is provided to the initial blood pressure determined according to the second PPG signal to calibrate the initial blood pressure, and the relatively accurate blood pressure obtained by calibration is used as the blood of the user. Thus, the blood pressure detection method of the embodiment of the present application is convenient to detect and has a relatively accurate blood pressure detection result. In addition, if a blood pressure value is determined only by the first PPG signal and the first pressure, the first pressure needs to act on the user every time the blood pressure is detected, which is cumbersome to operate and causes a bad experience for the user. In an embodiment of the present application, the blood pressure calibration information obtained by the previous processing can be acquire to calibrate the initial blood pressure currently obtained according to the second PPG signal, so there is no need for the first pressure to act on the user every time the blood pressure is detected, and the first pressure and the first PPG signal are detected. Therefore, the accuracy of blood pressure detection is further improved while the convenience of blood pressure detection and the user experience are improved.

In some possible implementation manners, the acquiring the blood pressure calibration information, includes: driving a pressure application module to apply a pressure to the user to form the first pressure; controlling a first pulse wave detection module to detect the first PPG signal when the first pressure acts on the user; controlling a pressure detection module to detect the first pressure; receiving the first pressure and the first PPG signal, and determining the blood pressure calibration information according to the first pressure and the first PPG signal.

In some possible implementation manners, the acquiring the blood pressure calibration information, includes: controlling a prompt module to output a prompt signal, the prompt signal configured to prompt the user to apply a pressure to the blood pressure detection apparatus to form the first pressure; controlling a first pulse wave detection module to detect the first PPG signal when the first pressure acts on the user; controlling a pressure detection module to detect the first pressure; receiving the first pressure and the first PPG signal, and determining the blood pressure calibration information according to the first pressure and the first PPG signal.

In some possible embodiments, the first pressure changes from large to small or from small to large with time.

In some possible implementation manners, the acquiring a second photo plethysmography (PPG) signal, and processing according to the second PPG signal to obtain the user's initial blood pressure, includes: controlling a second pulse wave detection module to detect the second PPG signal of the user; receiving the second PPG signal, and processing the second PPG signal by applying a pulse wave analysis method or a pulse transit time measurement method, to obtain the user's initial blood pressure.

In some possible implementation manners, the first pressure changes with time, the determining the blood pressure calibration information according to the first pressure and the first PPG signal, includes: sorting the first PPG signal corresponding to the first pressure according to a magnitude of the first pressure to form an envelope signal of the first PPG signal; determining the first blood pressure of the user according to the envelope signal; using the first blood pressure as the blood pressure calibration information.

In some possible implementation manners, the calibrating the initial blood pressure according to the blood pressure calibration information, and using the blood pressure obtained by calibration as the blood pressure of the user, includes: using the blood pressure calibration information as a direct current component of the blood pressure of the user; using the initial blood pressure as an alternating current component of the blood pressure of the user; calibrating the alternating current component of the blood pressure of the user according to the direct current component of the blood pressure of the user, and using the blood pressure obtained by calibration as the blood pressure of the user.

In some possible implementation manners, before the acquiring a second photo plethysmography (PPG) signal, the blood pressure detection method further includes: determining whether the user is in an exercise state; if not, acquiring the second PPG signal; if so, re-determining whether the user is in the exercise state after an interval preset time period.

In some possible implementation manners, the acquiring the first photo plethysmography (PPG) signal, includes: acquiring the first PPG signal at a first part of the user; the acquiring the second PPG signal, includes: acquiring the second PPG signal at a second part of the user, where the second part is different from the first part.

In some possible implementation manners, the first part is a finger of the user and the second part is a wrist of the user.

In the second aspect, a blood pressure detection apparatus is provided, including a processor, the processor configured to: acquire blood pressure calibration information, the blood pressure calibration information being information obtained by processing according to a first pressure acting on a user and a first photo plethysmography (PPG) signal when the first pressure acts on the user; acquire a second photo plethysmography (PPG) signal, and process according to the second PPG signal to obtain the user's initial blood pressure; calibrate the initial blood pressure according to the blood pressure calibration information, and use the blood pressure obtained by calibration as the blood pressure of the user.

In some possible implementation manners, the processor is configured to: drive a pressure application module to apply the first pressure to the user to form the first pressure; control a first pulse wave detection module to detect the first PPG signal when the first pressure acts on the user; control a pressure detection module to detect the first pressure; receive the first pressure and the first PPG signal, and determine the blood pressure calibration information according to the first pressure and the first PPG signal.

In some possible implementation manners, the blood pressure detection apparatus further includes: the pressure application module electrically connected to the processor and the first pulse wave detection module electrically connected to the processor.

In some possible implementation manners, the processor is configured to: control a prompt module to output a prompt signal, the prompt signal configured to prompt the user to apply a pressure to the blood pressure detection apparatus to form the first pressure; control a first pulse wave detection module to detect the first PPG signal when the first pressure acts on the user; control a pressure detection module to detect the first pressure; receive the first pressure and the first PPG signal, and determine the blood pressure calibration information according to the first pressure and the first PPG signal.

In some possible implementation manners, the blood pressure detection apparatus further includes: the prompt module electrically connected to the processor and the first pulse wave detection module electrically connected to the processor.

In some possible implementation manners, the first pressure changes from large to small or from small to large with time.

In some possible implementation manners, the processor is configured to: control a second pulse wave detection module to detect the second PPG signal of the user; receive the second PPG signal, and process the second PPG signal by applying a pulse wave analysis method or a pulse transit time measurement method, to obtain the user's initial blood pressure.

In some possible implementation manners, the blood pressure detection apparatus further includes: the second pulse wave detection module electrically connected to the processor.

In some possible implementation manners, the first PPG signal changes with a magnitude of the first pressure; and the processor is configured to: sort the first PPG signal according to a magnitude of the first pressure to form an envelope signal of the first PPG signal; determine the first blood pressure of the user according to the envelope signal; use the first blood pressure as the blood pressure calibration information.

In some possible implementation manners, the processor is configured to: use the blood pressure calibration information as a direct current component of the blood pressure of the user; use the initial blood pressure as an alternating current component of the blood pressure of the user; calibrate the alternating current component of the blood pressure of the user according to the direct current component of the blood pressure of the user, and use the blood pressure obtained by calibration as the blood pressure of the user.

In some possible implementation manners, the processor is configured as before acquiring the second photo plethysmography (PPG) signal, and the processor is further configured to: determine if the user is in an exercise state; if not, acquire the second PPG signal; if so, re-determine whether the user is in the exercise state after an interval preset time period.

In some possible implementation manners, the processor is configured to: acquire the first PPG signal at a first part of the user; acquire the second PPG signal at a second part of the user, where the second part is different from the first part.

In some possible implementation manners, the first part is a finger of the user and the second part is a wrist of the user.

In a third aspect, an electronic device is provided, including: the blood pressure detection apparatus in the second aspect or any one of the possible implementation manners of the second aspect.

In some possible implementation manners, the electronic device is a smart watch.

### BRIEF DESCRIPTION OF DRAWINGS

FIG. 1 is a structural diagram of an electronic device to which a biological information detection system in the present application is applicable.
FIG. 2 is a schematic structural diagram of an apparatus for acquiring a photo plethysmography pulse wave by using a photoelectric sensor.
FIG. 3 is a waveform feature diagram of a pulse wave.
FIG. 4 is a schematic diagram of a relative relationship among an ECG waveform, a PPG waveform, and a PTT.
FIG. 5 is a schematic flowchart of a blood pressure detection method according to an embodiment of the present application.
FIG. 6 is a schematic flowchart of another blood pressure detection method according to an embodiment of the present application.
FIG. 7 is a schematic diagram of a prompt signal according to an embodiment of the present application.
FIG. 8 is a schematic flowchart of another blood pressure detection method according to an embodiment of the present application.
FIG. 9 is a schematic flowchart of another blood pressure detection method according to an embodiment of the present application.
FIG. 10 is a waveform schematic diagram that a first PPG signal changes with a first pressure according to an embodiment of the present application.
FIG. 11 is a schematic flowchart of another blood pressure detection method according to an embodiment of the present application.
FIG. 12 is a schematic flowchart of another blood pressure detection method according to an embodiment of the present application.
FIG. 13 is a schematic structural diagram of a blood pressure detection apparatus according to an embodiment of the present application.
FIG. 14 is a schematic structural diagram of another blood pressure detection apparatus according to an embodiment of the present application.
FIG. 15 is a top view of a smart watch according to an embodiment of the present application.
FIG. 16 is a bottom view of the smart watch in FIG. 15.
Fig. 17 is a side view of the smart watch in FIG. 15.
FIG. 18 is a schematic structural diagram of another blood pressure detection apparatus according to an embodiment of the present application.
FIG. 19 is a bottom view of another smart watch according to an embodiment of the present application.

### DESCRIPTION OF EMBODIMENTS

Technical solutions in embodiments of the present application will be described hereinafter with reference to the accompanying drawings.

It should be understood that specific examples in embodiments of the present application are just for helping those skilled in the art better understand the embodiments of the present application, rather than for limiting the scope of the embodiments of present application.

It should be further understood that, in various embodiments of the present application, the sequence number of each process does not mean the order of execution, and the order of execution of each process should be determined by its function and internal logic, and should not set any limitation to the implementation process of the embodiments of the present application.

It should be further understood that the various implementation manners described in the present specification may be implemented separately or in combination, which is not limited by the embodiments of the present application.

Unless otherwise illustrated, all technical and scientific terms used in the embodiments of the present application have the same meaning as commonly understood by those skilled in the technical art to which the present application pertains. Terms used in the present application are merely for the purpose of describing specific embodiments, and are not intended to limit the scope of the present application. The term "and/or" used in the present application includes any and all combinations of one or more of the associated listed items.

The present application can be applied to a biological information detection system, including, but not limited to, a blood pressure detection system. The biological information detection system can be applied to various types of electronic devices. The electronic device can be a smart wearable device, a mobile phone, a tablet computer, a mobile medical device, etc., where the smart wearable device can include at least one of the following devices: a watch, a bracelet, an anklet, a necklaces, glasses or a head-mounted device; and a mobile medical device may include at least one of the following devices: a blood glucose monitoring device, a heart rate monitoring device, a blood pressure measurement device, a body temperature measurement device, etc. The embodiment of the present application does not limit this.

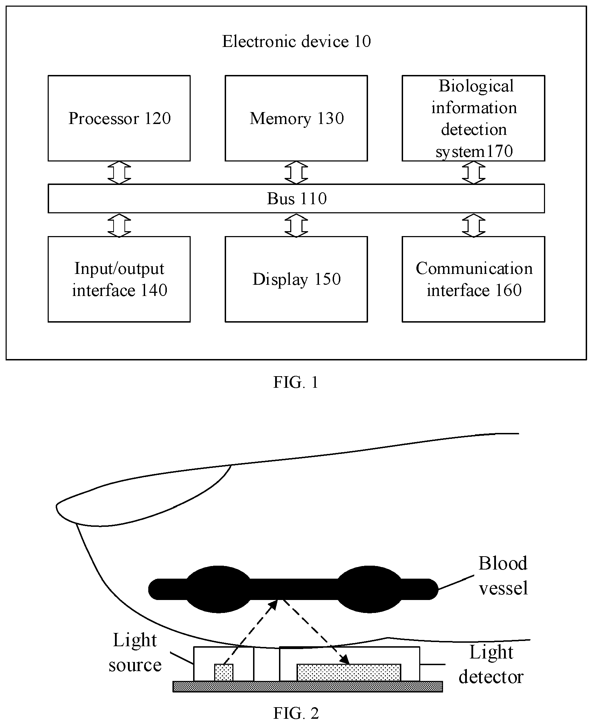

FIG. 1 shows a structural diagram of an electronic device to which a biological information detection system in the present application is applicable.

As shown in FIG. 1, an electronic device 10 may include a bus 110, a processor 120, a memory 130, an input/output interface 140, a display 150, a communication interface 160 and a biological information detection system 170.

The bus 110 may include a circuit that implements transmission and communication (for example, control messages or data) between components in the electronic device 10. The processor 120 may include one or more types of data processors for performing data processing. The memory 130 may include a volatile memory and/or a non-volatile memory. It may store an instruction or data related to other functional components in the electronic device 10.

The input/output interface 140 may be configured to receive the instruction or data, which is input from a user or an external device, and transmit to the other functional components in the electronic device 10, or may output an instruction or data, which is generated by the other functional components of the electronic device 10, to the user or the external device.

The display 150 may be, for example, a liquid crystal display (LCD), an organic light-emitting diode (OLED) display or a display of other types. The display 150 may display various types of contents (e.g., text, images, videos, icons, and the like) for a user. Further, the display 150 may include a touch screen, and the user may input related instruction information through the touch screen.

The communication interface 160 may be configured to implement communication between the electronic device 10 and an external device, for example, communication between a network server or other electronic devices. As an example, the communication interface 160 may be connected to a communication network through wireless or wired communication, and may communicate with an external device. Among them, wireless communication includes, but is not limited to, cellular communication or short-range communication. The wired communication may include but is not limited to one of a universal serial bus (USB), a high definition multimedia interface (HDMI), recommended standard 232 (RS-232), or other communication methods.

The biological information detection system 170 is configured to implement detecting the user's biometric information. The biometric information includes, but is not limited to: the user's heart rate, blood oxygen saturation, blood pressure and other parameter information, which can be obtained by testing the user's pulse wave. In other words, the biological information detection system 170 in the embodiment of the present application can be configured to detect the pulse wave of the user, and obtain one or more types of biometric information of the user based on calculation and analysis of the pulse wave.

In some embodiments, the electronic device 10 may omit at least one of the above components, or may further include other components, which will not be described in detail here.

Specifically, the embodiment of the present application relates to a blood pressure detection method and a blood pressure detection apparatus, which can be applied to the biological information detection system 170 in FIG. 1 and arranged in the electronic device 10 in FIG. 1. More specifically, the embodiment of the present application relates to a cuffless pulse wave-based blood pressure detection method and blood pressure detection apparatus, which has the advantages of portability, continuous non-invasive measurement, and high measurement accuracy.

In order to facilitate understanding, firstly, the related concepts involved in the present application are described.

### (1) Pulse wave.

A pulse wave refers to periodic fluctuation of an arterial wall caused by a periodic change in pressure and volume in an artery during cyclical systole and diastole of a heart, that is, a cyclical heartbeat pushes blood to run along a blood vessel to produce the pulse wave. Therefore, the pulse wave is not only affected by a functional state of the heart, but also by vascular resistance, vascular elasticity and blood viscosity in the artery flowing through various levels. A change in a physiological feature of the cardiovascular system will cause a change in intensity, shape, rhythm and speed of a pulse wave signal. Therefore, a feature of pulse wave signal can be analyzed and studied, and physiological and pathological information contained therein can be extracted to provide help for early diagnosis and prevention of a cardiovascular system related disease.

A photo plethysmography pulse wave is generally detected and acquired by a photoelectric sensor. It is usually detected and obtained by adopting a photo plethysmography (PPG) method. A curve of a blood volume changed with time obtained by adopting the method of tracing is a photo plethysmography pulse wave waveform diagram, and the photo plethysmography pulse wave will also be written as a photo plethysmography signal or PPG signal in the following.

Specifically, FIG. 2 shows a schematic structural diagram of an apparatus for acquiring a photo plethysmography pulse wave by using a photoelectric sensor.

As shown in FIG. 2, when a light source emits a light beam of a certain wavelength to irradiate a surface of human skin (such as a finger skin shown in FIG. 2), during each heartbeat systole and expansion of the blood vessel will affect light transmission (for example, in a transmission PPG, light passing through a fingertip) or light reflection (for example, in reflective PPG, light from near a surface of the finger). When the light passes through a skin tissue and then is reflected to the light detector, the light will be attenuated to a certain extent. Absorption of light by a muscle, a bone, a vein and other connecting tissues is basically unchanged (if there is no significant movement amplitude of a measured part), but the artery will be different. Since there is blood pulsation in the artery, the absorption of light will naturally change. Therefore, after the light detector converts the light signal reflected and/or transmitted by a human body into an electrical signal, since absorption of the light signal by the artery changes while the absorption of the light signal by other tissues is basically unchanged, the obtained signal can be divided into a direct current (DC) signal and an alternating current (AC) signal, from which an AC signal is extracted to reflect a feature of blood flow.

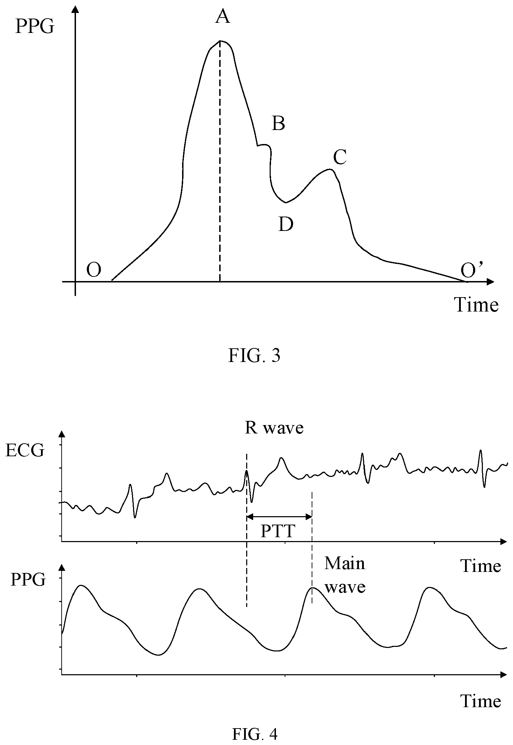

FIG. 3 shows a waveform feature diagram of a photoplethysmography pulse wave.

As shown in FIG. 3, a complete pulse wave waveform has 4 important feature points of A, B, C, and D, which include an ascending branch and a descending branch. As shown in FIG. 3, A is referred to as a main wave, B is referred to as a tidal wave, C is referred to as a dicrotic peak, D is referred to a dicrotic trough, OA is the an ascending branch of the main wave, and OO' is a pulse wave cycle.

The OA segment is the ascending branch of the pulse waveform, which is due to systole and ejection of the left ventricle and a rapid increase in an arterial blood pressure, resulting in expansion of the arterial wall. The point O is the starting point of an ejection period of the heart, and the point A is the highest point of the aortic pressure, reflecting the maximum value of the pressure and volume in the artery.

The AD segment is an anterior segment of the descending branch of the pulse waveform, which is due to the process that an ejection velocity begins to decrease at the later stage of the ventricular ejection, causing a blood volume flowing from the aorta to the surrounding to be greater than a blood volume flowing into the aorta, and the artery changes from expansion back to retraction, and the arterial blood pressure gradually decreases. The point B is the stop point of ventricular ejection of the left ventricle. It is the peak point of a reflected wave, also referred to as a tidal wave peak, which reflects tension, compliance and peripheral resistance of an artery blood vessel. The point D is a trough point of the tidal wave, that is, a dividing point between systole and diastole of the heart.

The DO' segment is the posterior segment of the descending branch of the pulse waveform, also referred to as a dicrotic wave. It is formed due to ventricular diastole, and the arterial blood pressure continues to decrease, and the blood in the aorta flows back toward the direction of the ventricle. The functional status of the aorta, blood vessel elasticity and blood flow state are reflected.

### (2) A pulse transit time (PTT) measurement method and a pulse wave velocity (PWV) measurement method.

Specifically, PTT refers to a conduction time of the pulse wave from the heart to a measurement site during arterial ejection. PWV can be calculated by the PTT based on a positional relationship between the measurement site and the heart. In the existing technical theory, the PWV has a linear relationship with the blood pressure, and the blood pressure can be calculated and obtained according to the PWV or the PTT and a related data model.

At present, since the pulse wave velocity PWV is difficult to detect, existing PWV-based blood pressure detection methods all rely on the detection of the PTT.

In some methods, a blood pressure detection technology combining the PPG by electrocardiography (ECG) can be configured to detect and obtain the PTT and thereby estimating and obtaining the blood pressure.

FIG. 4 shows a relative relationship among an ECG waveform, a PPG waveform, and a PTT.

As shown in FIG. 4, in the ECG waveform, an R wave represents systole of the ventricle. The time interval between the R wave in the ECG waveform and the main wave in the PPG waveform can be expressed as PPT. Alternatively, the PPT can also be expressed by a time interval before other feature points by an R waveform to the PPG waveform in the ECG waveform.

Specifically, using this method to detect and obtain the blood pressure as a high-frequency component of a systolic blood pressure (SBP) according to the PTT and a function equation, a low-frequency component of systolic blood pressure requires a more accurate blood pressure measurement method, such as obtained by the auscultation method or the oscillometric method. A final relatively accurate value of systolic blood pressure can be determined based on the sum of the low-frequency component and high-frequency component of the systolic blood pressure. Further, according to the function equation among PTT, the more accurate systolic blood pressure (SBP) and the diastolic blood pressure (DPB), a relatively accurate value of the diastolic blood pressure (DPB) can be obtained. In other words, to use this method, it needs to periodically measure and obtain an accurate low-frequency component of blood pressure through an external device such as a sphygmomanometer, and calibrate the blood pressure detected and obtained according to a PPT detection method, so as to obtain a more accurate blood pressure detection result.

### (3) Pulse Wave Analysis (PWA).

Specifically, a feature parameter in the pulse wave can be extracted. By analyzing correlation between the feature parameter and the blood pressure, the feature parameter with the best correlation with blood pressure can be found, which is used as a variable for measuring the blood pressure. Then regression analysis is performed to establish a regression function equation for blood pressure measurement.

Optionally, an amplitude of any feature point or a time difference between any two feature points in the pulse wave shown in above FIG. 3 can be adopted as the feature parameter. And correlation analysis can be performed on the feature parameter and the blood pressure through a large amount of experimental data, to establish and obtain the regression function equation. In an actual blood pressure measurement process, the pulse wave is detected, from which the feature parameter is extracted, and then the blood pressure is measured according to the feature parameter and the regression function equation.

The blood pressure detected and obtained by the method is calculated and obtained by the regression function equation. There is a parameter obtained by artificial fitting in the regression function equation. With time changes and human body differences, a blood pressure measurement result calculated and obtained by the regression function equation may have a certain error. Therefore, adopting the method also requires regular calibration of the blood pressure measurement result, that is, it needs to periodically measure and obtain the accurate blood pressure through an external device such as a sphygmomanometer, calibrate the blood pressure detected and obtained according to the PWA detection method or calibrate the parameter in the regression equation, so as to obtain a more accurate blood pressure detection result. Therefore, based on the above description, it can be known that although the pulse wave can be obtained by adopting the above cuffless blood pressure detection method, so as to obtain the blood pressure by the pulse wave detection, to realize that the blood pressure detection apparatus can be carried around. However, the above cuffless blood pressure detection methods also have the problem of inaccurate blood pressure measurement and cumbersome calibration.

Based on this, the present application provides a cuffless blood pressure detection method and apparatus, which has the advantages of convenience, continuous non-invasive measurement, and high measurement accuracy.

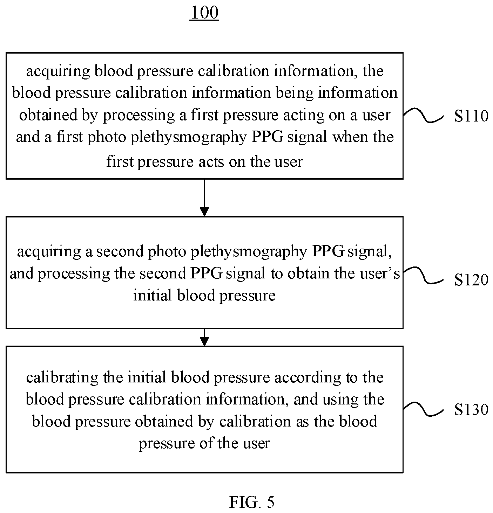

FIG. 5 shows a schematic flowchart of a blood pressure detection method 100 provided by the present application. The blood pressure detection method 100 is applied to a blood pressure detection apparatus. The blood pressure detection apparatus may be the blood pressure detection apparatus for detecting blood pressure information in the biological information detection system 170 of FIG. 1. The blood pressure detection apparatus includes a processor. The processor is an execution body of the blood pressure detection method 100 described below.

As shown in FIG. 5, the blood pressure detection method 100 includes the following steps.

S110: acquiring blood pressure calibration information, the blood pressure calibration information being information obtained by processing a first pressure acting on a user and a first photo plethysmography (PPG) signal when the first pressure acts on the user.

S120: acquiring a second photo plethysmography (PPG) signal, and processing the second PPG signal to obtain the user's initial blood pressure.

S130: calibrating the initial blood pressure according to the blood pressure calibration information, and using the blood pressure obtained by calibration as the blood pressure of the user.

Specifically, in step S110, the processor acquires the first pressure that acts on the user and the first photo plethysmography (PPG) signal when the first pressure acts on the user at the current time, and the processor obtains the blood pressure calibration information according to the first pressure and the first PPG signal. Alternatively, the processor directly calls the blood pressure calibration information obtained at the previous time.

When the first pressure acts on the user, a blood vessel in a pressured part is deformed, where the blood volume changes. Therefore, the first PPG signal changes with a change of the first pressure. Therefore, the blood pressure calibration information may be determined according to the relationship that the first PPG signal changes with the first pressure.

In step S120, when no pressure acts on the user, the processor may acquire the second PPG signal of the user, and process the second PPG signal to obtain the initial blood pressure. Detection of the second PPG signal will not affect the user, can be acquired for a long time and continuously, and is non-invasive detection. However, since the second PPG signal does not include pressure change information, accuracy of the initial blood pressure obtained by processing the second PPG signal is low. If the initial blood pressure is directly provided to the user, the user experience will be affected.

In step S130, the above initial blood pressure is calibrated according to the above blood pressure calibration information, and a relatively accurate blood pressure after calibration can be obtained. The blood pressure obtained by calibration is used as a current blood pressure of the user and fed back to the user.

In the technical solution of the embodiment of the present application, the acquired second PPG signal is a PPG signal detected and obtained when no pressure acts on the user's body surface, and the accuracy of the initial blood pressure obtained based on the second PPG signal is relatively low. At this time, there is no need to resort to an external assistance device such as a sphygmomanometer for calibration, but to directly measure and obtain the first PPG signal and the first pressure according to the blood pressure detection apparatus to determine the blood pressure calibration information, the blood pressure calibration information is provided to the initial blood pressure, the blood pressure calibration information is used to calibrate the initial blood pressure to obtain a relatively accurate blood pressure after calibration, which is used as the current blood pressure of the user and fed back to the user. Thus, the blood pressure detection method provided by the embodiment of the present application does not require to adopt assistance from other apparatuses additionally, is convenient to operate, and can obtain a relatively accurate blood pressure detection result. In addition, if a blood pressure value is determined only by the first PPG signal and the first pressure, the first pressure needs to act on the user every time the blood pressure is detected, which is cumbersome to operate and causes a bad experience for the user. In an embodiment of the present application, the blood pressure calibration information obtained by the previous processing can be acquired to calibrate the initial blood pressure currently obtained according to the second PPG signal, so there is no need for the first pressure to act on the user every time the blood pressure is detected and the first pressure and the first PPG signal are detected. Therefore, the accuracy of blood pressure detection is further improved while the convenience of blood pressure detection and the user experience are improved.

Optionally, in the above step S 110, the first PPG signal at the first part of the user may be acquired, and in the above step S120, the second PPG signal at the second part of the user may be acquired.

Combined with the related description of FIG. 2 above, it can be understood that the blood pressure detection apparatus or the electronic device where it is located includes a PPG detection module, and the PPG detection module includes a light source and a light detector for detecting the first PPG signal and the second PPG signal.

In some implementation manners, the first part and the second part are different parts of the user. In this case, the blood pressure detection apparatus or the electronic device where it is located includes light sources and light detectors corresponding to two different parts, that is, a first light source and a first light detector corresponding to the first part of the user, for detecting the first PPG signal at the first part; and a second light source and a second light detector corresponding to the second part of the user, for detecting the second PPG signal at the second part.

As an example, the first part of the user may be a finger of the user, such as an index finger, and the above first light source and the first light detector may be controlled to detect the first PPG signal at the finger. The second part of the user may be a wrist of the user, and the above second light source and the second light detector can be controlled to detect the second PPG signal at the wrist. In this case, the blood pressure detection apparatus of the embodiment of the present application can be arranged in a smart watch. The above first light source and the first light detector can be correspondingly arranged on a side of the smart watch, so as to help the user to detect the first pressure and the first PPG signal by pressing with the finger, to obtain the blood pressure calibration information. The above second light source and the second light detector can be correspondingly arranged on the back of the smart watch to detect the second PPG signal at the wrist, which can help the user to perform blood pressure detection. For example, the second light source and the second light detector located on the back can facilitate long-term detection of the blood pressure of the user (that is, the user does not need to press the watch for a long time), so as to monitor the blood pressure of the user for a long time, such as continuous blood pressure detection and monitoring for a long time can also be performed conveniently when the user is in a sleeping state. Then, the blood pressure calibration information obtained by the first pressure and the first PPG signal at the finger is used to calibrate the blood pressure obtained by the second PPG signal at the wrist, so that convenient blood pressure detection can be realized and the blood pressure detection result is relatively accurate.

Of course, the first part and the second part can also be the same part of the user. For example, the first part and the second part are both the wrist of the user or are both other parts of the user. In this case, the blood pressure detection apparatus or the electronic device where it is located may only include a light source and a light detector corresponding to one part, that is, the electronic device may only include a light source and a light detector for detecting the first PPG signal when the first pressure acts on the user and the second PPG signal when no pressure acts on the user at different times. If the blood pressure detection apparatus is arranged in the smart watch, the one light source and the one light detector can be correspondingly arranged on the back of the smart watch.

But preferably, the first part and the second part are different parts of the user, respectively, for the following reasons.

First, by testing PPG signals of different parts of the user, and combining the PPG signals of different parts to determine the blood pressure of the user, the accuracy of the blood pressure test can be improved.

Secondly, if the first part is the finger and the second part is the wrist, the wrist has a smaller movement amplitude relative to the finger; and the wrist is suitable for a wearable smart terminal device, such as wearing a smart watch. The second PPG signal comes from the wrist, which makes the wearable device more suitable for long-term continuous blood pressure detection, thereby improving the user experience.

Further, the first part needs applying a pressure to it. The finger per se is convenient for pressure operation, and the skin of the finger is thinner than the skin of the wrist. When a pressure signal acts on the finger, the first PPG signal generated by it changes significantly with the first pressure, and can detect and obtain the relatively accurate blood pressure calibration information. The blood pressure calibration information is provided to the second PPG signal generated at the wrist, which can improve the accuracy of the blood pressure tested at the wrist.

Optionally, the above first pressure may act on the user in a variety of different ways.

For example, in the first implementation manner, the above first pressure is a pressure signal generated by the user per se. As an example, it may be a pressure signal generated by applying a pressure to the blood pressure detection apparatus by the first part of the user. For example, the finger of the user applies a pressure to the blood pressure apparatus, and the blood pressure detection apparatus will correspondingly apply a reaction force to the finger of the user, to form the first pressure.

For another example, in the second implementation manner, the above first pressure is a pressure signal generated by the blood pressure detection apparatus. As an example, it may be a pressure signal generated by applying a pressure to the first part of the user by the blood pressure detection apparatus. For example, the blood pressure detection apparatus per se applies the pressure signal to the finger of the user.

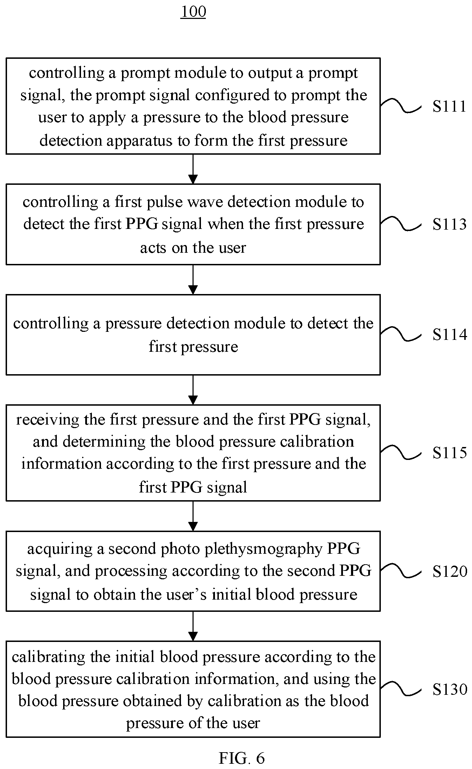

Hereinafter, an execution process of the blood pressure detection method in the above two implementation manners is described with reference to FIG. 6 to FIG. 8.

FIG. 6 shows a schematic flowchart of a blood pressure detection method 100 in the above first implementation manner.

As shown in FIG. 6, the above step S110 includes the following steps.

S111: controlling a prompt module to output a prompt signal, the prompt signal configured to prompt the user to apply a pressure to the blood pressure detection apparatus to form the first pressure.

S113: controlling a first pulse wave detection module to detect the first PPG signal when the first pressure acts on the user.

S114: controlling a pressure detection module to detect the first pressure.

S115: receiving the first pressure and the first PPG signal, and determining the blood pressure calibration information according to the first pressure and the first PPG signal.

Optionally, the prompt signal includes, but is not limited to, one or more of a text signal, an image signal, a sound signal, a vibration signal, or a light signal, and is intended to be configured to interact with the user. Correspondingly, in order to control the prompt module to output the above different types of prompt signals, the blood pressure detection apparatus or the electronic device where the blood pressure detection apparatus is located may include different types of prompt modules.

For example, after receiving the prompt signal, the user applies a pressure to the blood pressure detection apparatus by the first part, and the reaction force of the blood pressure detection apparatus on the first part forms the first pressure on the first part. At this time, the processor controls the first pulse wave detection module in the blood pressure detection apparatus or in the electronic device where the blood pressure detection apparatus is located to detect the first PPG signal when the first pressure acts on the user, and controls the pressure detection module in the blood pressure detection apparatus or in the electronic device where the blood pressure detection apparatus is located to detect the first pressure.

Then, the processor receives the first PPG signal transmitted by the first pulse wave detection module and the first pressure transmitted by the pressure detection module, and determines the blood pressure calibration information according to the first pressure and the first PPG signal.

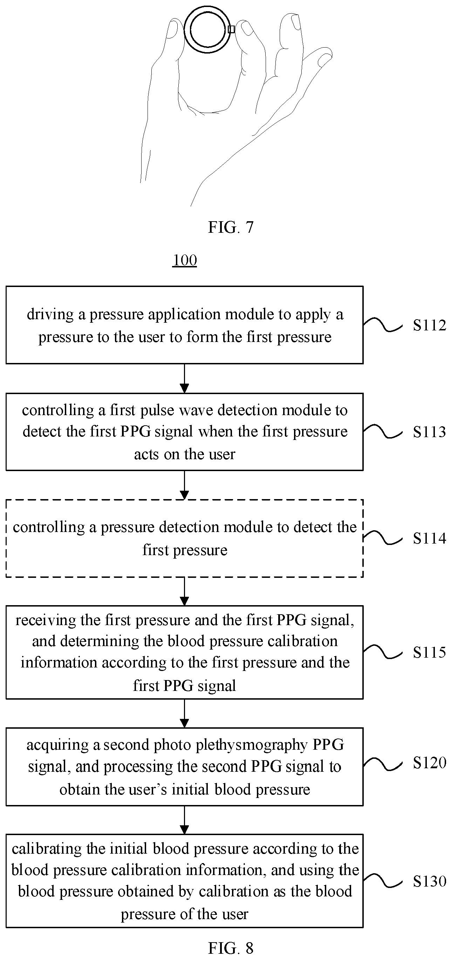

Optionally, the above prompt signal is an image signal or a video signal, which can show the user a specific pressing method. For example, at least part of a functional module in the blood pressure detection apparatus, such as the first pulse wave detection module, is arranged on the side of the smart watch, and the prompt signal can prompt the user to press in the pressing method shown in FIG. 7, where the index finger of the user is pressed on one side of the watch, and the thumb is pressed on another side of the watch. The first pulse wave detection module can be arranged below the thumb or index finger. Adopting the pressing method can improve stability of pressing, thereby improving detection accuracy of the first pressure and detection accuracy of the first PPG signal.

Further, if the finger of the user is pressed on the blood pressure detection apparatus, that is, when the first pressure acts on the finger of the user, at this time, the first light source in the first pulse wave detection module is controlled to turn on, to emit a first light signal to the finger of the user. After the first light signal is reflected and/or transmitted by the finger of the user, it is received by the first light detector in the first pulse wave detection module to form the first PPG signal.

Optionally, in the embodiment of the present application, the first light source may emit a light signal of a target waveband. The target waveband includes, but is not limited to, a red light waveband or a green light waveband, and is configured to obtain the first PPG signal of a good signal quality.

Optionally, the prompt signal can also be configured to prompt the user a method of applying a pressure, for example, increasing the applied pressure or decreasing the applied pressure. After receiving the prompt signal, the user adjusts a pressing force with time. For example, first apply a pressure to the blood pressure detection apparatus with a small pressing force, and then gradually increase the pressing force. For another example, first apply a pressure to the blood pressure detection apparatus with a large pressing force, and then gradually decreases the pressing force. Alternatively, the pressing force can also be changed in any other manner, which is not specifically limited in the embodiment of the present application.

Further, when the pressing force of the finger of the user changes, the first pressure acting on the finger of the user changes with time, and the blood volume in the blood vessel inside the finger also changes with time. Therefore, a waveform of the first PPG signal changes with time, and corresponds to different first pressures.

FIG. 8 shows a schematic flowchart of another blood pressure detection method 100 in the above second implementation manner.

As shown in FIG. 8, the above step S110 includes the following steps.

S 112: driving a pressure application module to apply a pressure to the user to form the first pressure.

S113: controlling a first pulse wave detection module to detect the first PPG signal when the first pressure acts on the user.

S115: receiving the first pressure and the first PPG signal, and determining the blood pressure calibration information according to the first pressure and the first PPG signal.

Optionally, the blood pressure detection apparatus or the electronic device where it is located includes a pressure application module, and the pressure application module can be configured to apply a pressure to the user. As an example, in the embodiment of the present application, the processor may drive the pressure application module to apply a pressure to the user to form the first pressure. The pressure application module includes, but is not limited to, a motor drive unit or other types of drive unit, which is not specifically limited in the embodiment of the present application.

Further, when the pressure application module is driven to apply a pressure to the first part of the user, such as a finger, the processor controls to turn on the first light source in the first pulse wave detection module, and emits the first light signal to the finger of the user. After the first light signal is reflected and/or transmitted by the finger of the user, it is received by the first light detector in the first pulse wave detection module to form the first PPG signal.

Optionally, in some implementation manners, the pressure application module may directly send the pressure that it applies to the user as the first pressure to the processor.

Alternatively, in some other implementation manners, the blood pressure detection apparatus or the electronic device where it is located may include a pressure detection module. Optionally, in the blood pressure detection method 100 shown in FIG. 8, before step S115, step S114 may also be included (the dashed box in FIG. 8 indicates that this step is optional): controlling the pressure detection module to detect the first pressure. Specifically, the processor may control the pressure detection module to detect the pressure applied by the pressure application module to the user, to obtain the first pressure and send it to the processor.

Optionally, a pressure intensity applied by the pressure application module to the finger of the user may change with time. For example, the pressure intensity changes from small to large or from large to small with time, or it can also change in any other manner, which is not specifically limited in the embodiment of the present application.

Further, when the pressure signal applied by the pressure application module to the finger of the user changes, the first pressure acting on the finger of the user changes with time, and the blood volume in the blood vessel inside the finger also changes with time. Therefore, the waveform of the first PPG signal changes with time, and corresponds to different first pressures.

Adopting the technical solution of the embodiment of the present application, by controlling the pressure application module to apply a pressure to the user to form the first pressure, it can avoid the need for the user to actively press when the blood pressure is detected, thereby improving the user experience. Moreover, compared to the user's pressing pressure, by controlling the pressure application module to provide the user with the first pressure, it can also prevent the user's pressing from damaging the blood pressure detection apparatus and the electronic device where the blood pressure detection apparatus is located.

From the above description of FIG. 6 and FIG. 8, it can be seen that a magnitude of the first pressure acting on the user changes with time. Therefore, the detected first PPG signal changes with the magnitude of the first pressure.

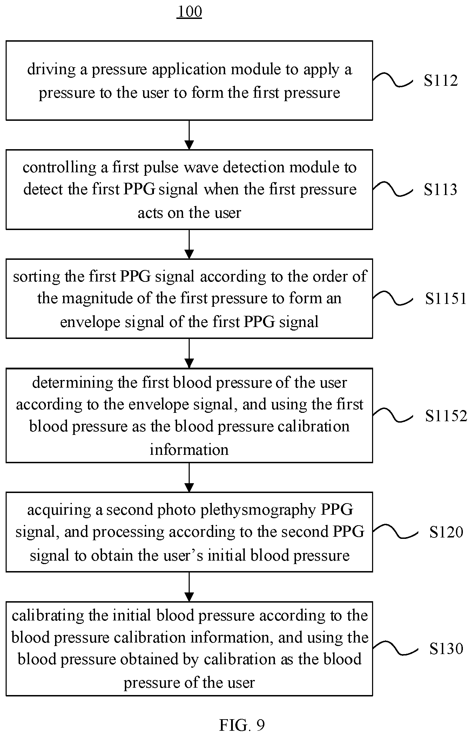

In this case, FIG. 9 shows a schematic flowchart of another blood pressure detection method 100.

As shown in FIG. 9, the above step S110 includes the following steps.

S1151: sorting the first PPG signal according to the order of the magnitude of the first pressure to form an envelope signal of the first PPG signal.

S1152: determining the first blood pressure of the user according to the envelope signal, and using the first blood pressure as the blood pressure calibration information.

Optionally, in step S1151, the first PPG signal corresponding to different pressures are sorted according to the order of the first pressure from large to small or the order from small to large, to form the envelope signal of the first PPG signal. According to the order of the first pressure, the envelope signal of the first PPG signal can more accurately reflect the relationship between an amplitude of the PPG signal and the change of the first pressure. Compared with the envelope signal acquired according to other methods, the envelope signal formed in this way has a good signal quality. The blood pressure detected and obtained according to the envelope signal has a high accuracy.

It is understandable that step S1151 and step S1152 in FIG. 9 are applied to the embodiment of the blood pressure detection method 100 shown in FIG. 8, and step S1151 and step S1152 can also be applied to the embodiment of the blood pressure detection method 100 shown in FIG. 6.

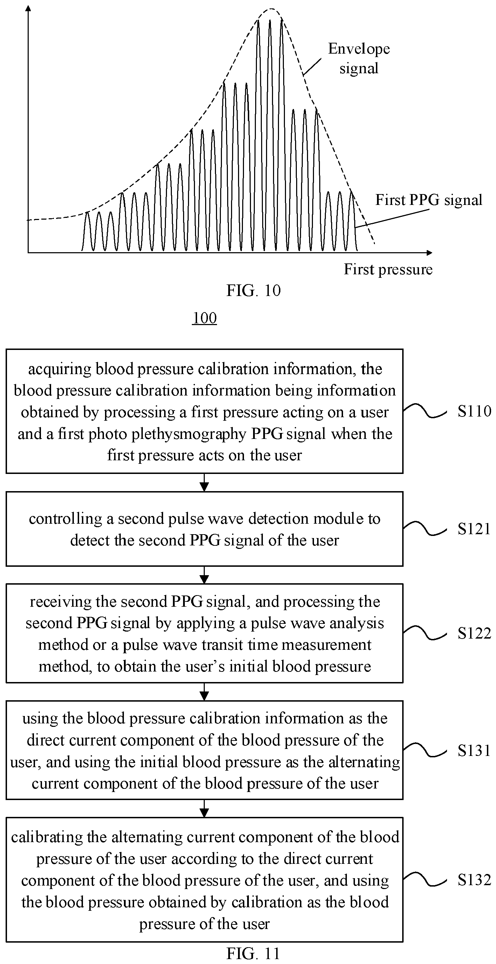

FIG. 10 shows a change trend of the corresponding first PPG signal when the first pressure changes from small to large.

It can be seen from FIG. 10 that as the first pressure gradually increases, an amplitude of the first PPG signal gradually increases. When the amplitude reaches the maximum, the first pressure is increased, then the amplitude of the first PPG signal gradually decreases until it disappears. Therefore, an amplitude of the envelope signal formed by the first PPG signal first increases with the pressure, and gradually decreases to zero as the pressure signal increases after reaching the maximum value.

In step S1152, as an embodiment, the first blood pressure of the user can be determined according to a waveform parameter of the envelope signal and a preset function equation. For example, a current diastolic blood pressure and a systolic blood pressure of the user are determined, and the first blood pressure can be directly used as the blood pressure calibration information.

Optionally, the above preset function equation may be a function equation determined and obtained according to a plurality of sets of experimental data, so as to improve calculation reliability of blood pressure calculation through the function equation and the waveform parameter of the envelope signal.

In order to ensure the accuracy of the blood pressure calibration information, optionally, the following steps may be performed before the above step S1152:
determining whether the above envelope signal meets a preset condition, if so, performing the above step S1151, if not, following the process of steps S111 and S113 shown in FIG. 6 or the process of steps S112 and S113 shown in FIG. 8, and re-detecting a new PPG signal generated under a new pressure signal.

Specifically, judging whether the envelope signal meets the preset condition is judging a signal quality of the envelope signal. If the signal quality of the envelope signal is poor, it indicates a change trend is not obvious that the detected first PPG signal changes with the first pressure. The first blood pressured detected and obtained according to the envelope signal is inaccurate, that is, the blood pressure calibration information is inaccurate.

Optionally, in the embodiment of the present application, the above preset condition of the envelope signal includes, but is not limited to: integrity of the envelope signal, the maximum amplitude of the envelope signal, a width of the envelope signal, etc., which is not specifically limited in the embodiment of the present application.

Optionally, in the above steps, if the envelope signal does not meet the preset condition, it is determined whether the number of times of failing to meet exceeds a threshold value. If it exceeds the threshold value, a blood pressure detection process is ended; and if it does not exceed the threshold value, the process of the above step S110 is followed, the new PPG signal generated under the new pressure signal is re-detected.

Adopting this method, it can avoid repeating the calibration process under a poor test condition, which wastes system resources and brings a bad experience to the user.

FIG. 11 shows a schematic flowchart of another blood pressure detection method 100.

As shown in FIG. 11, as a possible implementation manner, the above step S120 may include the following steps.

S121: controlling a second pulse wave detection module to detect the second PPG signal of the user.

S122: receiving the second PPG signal, and processing the second PPG signal by applying a pulse wave analysis method or a pulse wave transit time measurement method, to obtain the user's initial blood pressure.

Optionally, in step S121, the second light source in the second pulse wave detection module is controlled to turn on at a target frequency within a preset time period. The light signal reflected and/or transmitted by the user is received by the second light detector in the second pulse wave detection module, to detect the second PPG signal.

As an example, the preset time period is 1s, the target frequency is 50Hz, and the second light source is turned on for a plurality of times at a frequency of 50Hz, that is, the first light source is turned on every 20ms, so as to detect and obtain the second PPG signal within 1s.

Of course, the preset time period and the target frequency may also be any other preset values, which are not specifically limited in the embodiment of the present application. It can be understood that the longer the preset time period, the higher the target frequency, the higher the detection accuracy of the second PPG signal.

Optionally, in the embodiment of the present application, the second light source may emit a light signal of a target waveband. The target waveband includes, but is not limited to, a red light waveband or a green light waveband, and is configured to obtain the second PPG signal of a good signal quality.

Optionally, in addition to adopting step S121 of controlling the second pulse wave detection module to detect the second PPG signal of the user, the above first pulse wave detection module may further be controlled to detect the second PPG signal of the user. That is, the first pulse wave detection module is controlled to detect the first PPG signal and the second PPG signal of the same part of the user in different time periods, respectively, where the first PPG signal is a PPG signal when the first pressure acts on the user, and the second PPG signal is a PPG signal when no pressure acts on the user.

Further, as shown in FIG. 11, after the second PPG signal is detected and obtained by step S121, in step S122, the above PTT measurement method or PWA analysis method may be adopted to process the second PPG signal to obtain the user's initial blood pressure. Alternatively, other related technical methods may be adopted to obtain the user's initial blood pressure according to the second PPG signal, which is not specifically limited in the embodiment of the present application.

Specifically, if the PTT measurement method is adopted to measure the blood pressure, it further needs to measure an ECG signal corresponding to the second PPG signal, and determine the PTT according to the ECG signal and the second PPG signal, so as to determine the alternating current component of the current blood pressure of the user, that is, the initial blood pressure.

Optionally, as shown in FIG. 11, the above step S130 may include the following steps.

S131: using the blood pressure calibration information as the direct current component of the blood pressure of the user, and using the initial blood pressure as the alternating current component of the blood pressure of the user.

S132: calibrating the alternating current component of the blood pressure of the user according to the direct current component of the blood pressure of the user, and using the blood pressure obtained by calibration as the blood pressure of the user.

In the implementation manner, the blood pressure calibration information acquired in step S 110 may be the above first blood pressure. In step S131 and S132, the first blood pressure is used as the direct current component of the current blood pressure of the user, and the initial blood pressure is used as the alternating current component of the blood pressure of the user. According to the first blood pressure and the initial blood pressure, the current blood pressure of the user is determined and fed back to the user as an output.

Alternatively, in some other implementation manners, in the process of processing the second PPG signal by adopting the above PTT measurement method or PWA analysis method, the function equation is used to calculate the blood pressure. According to the above blood pressure calibration information, such as the first blood pressure, a function equation parameter in the process of processing the second PPG signal is calibrated, so that the initial blood pressure calculated and obtained according to the second PPG signal and the calibrated function equation is close to or even equal to the first blood pressure. The first blood pressure or the initial blood pressure is used as the current blood pressure of the user, and output and feedback to the user.

The blood pressure detected and obtained by the method is more accurate, and can realize long-term and continuous blood pressure measurement, and can improve the user experience.

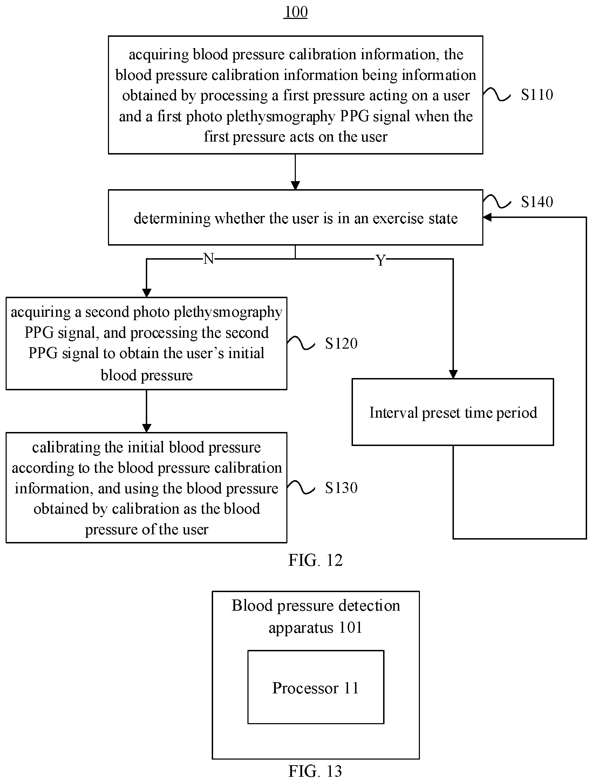

On the basis of the blood pressure detection method 100 shown in the above FIG. 5, FIG. 12 shows a schematic diagram of another blood pressure detection method 100.

As shown in FIG. 12, the blood pressure detection method 100 may further include:
S140: determining whether the user is in an exercise state.

If no, performing step S 120 and step S130, acquiring the second PPG signal, processing the second PPG signal to obtain the user's initial blood pressure, and determining the blood pressure of the user according to the blood pressure calibration information and the initial blood pressure.

If so, not performing step S 120 and step S 130, re-performing step S 140 after an interval preset time period, that is, re-determining if the user is in the exercise state.

Since the relative movement between an optical sensor (including the light source and the light detector) and the skin will reduce sensitivity of the light signal, if the user is in an exercise state, it will cause great interference to the detection of the second PPG signal. Therefore, before acquiring the second PPG, a user's state is first determined, and the second PPG signal when the user is not in an exercise state is detected and acquired, which can improve the accuracy of blood pressure detection.

Optionally, the user's state can be sensed by a motion sensor. The motion sensor includes, but is not limited to, an accelerometer. If the user is in a stationary state, three axes of XYZ in the accelerometer all have acceleration of 0. The second PPG signal detected and obtained at this time has the best quality.

In some implementation manners, whether the user is in an exercise state can be determined by determining whether the acceleration of the three axes of XYZ in the accelerometer is within a preset threshold value. The preset thresholds may all be 0, or at least one of them may be 0, or may be other preset thresholds, which is not specifically limited in the embodiment of the present application.

Various blood pressure detection methods provided by the embodiment of the present application are described above with reference to FIG. 5 to FIG. 12., The methods use the same blood pressure detection apparatus to detect and obtain different PPG signals in two detection methods during a blood pressure detection process, and combines results obtained in the two detection methods to jointly determine the blood pressure of the user. Convenient blood pressure detection is realized while the accuracy of blood pressure detection is improved at the same time.

In addition, it should be described that when the first PPG signal is detected above, the first pressure is required to act on the user's body surface. In a measurement process, the first pressure is a pressure signal that acts on the user in a short period of time. The blood pressure measured by this method is relatively accurate but not suitable for long-term or frequent blood pressure detection. Long-term or frequent pressure applied on the user's body surface will cause a poor user experience.

In order to solve the above problem, the embodiment of the present application provides another blood pressure detection method. On the basis of the above blood pressure detection method 100, that is, on the basis of determining the blood pressure calibration information, in the subsequent blood pressure detection process, before the blood pressure detection, first whether the calibration is updated at present is determined. If the calibration is updated, new blood pressure calibration information is re-acquired. The new blood pressure calibration information is new blood pressure calibration information obtained by processing according to the first PPG signal when the first pressure acts on the user again and the re-applied first signal. In addition, the new second PPG signal when no pressure acts on the user is re-acquired, a new initial blood pressure is determined, and a new blood pressure of the user is determined by calibrating the new initial blood pressure in combination with the new blood pressure calibration information. If the calibration is not updated, the previous blood pressure calibration information is directly called to acquire the new second PPG signal when no pressure acts on the user. Moreover, after the new initial blood pressure is determined, the new initial blood pressure is calibrated directly based on the previous blood pressure calibration information, to determine the new blood pressure of the user.

As an implementation manner, before acquiring the blood pressure calibration information, whether to perform calibration may be determined according to a first information. The first information includes, but is not limited to: current time information and/or user input information.

Specifically, if the first information is the current time information, whether the current time information is within the preset time range is determined. If the current time information is within the preset time range, calibration is performed. Otherwise, if the time information is not within the preset time range, no calibration will be performed.

As an example, the preset time range may be a time period preset by the user, such as the same time period every month, or a daily time period, or any other preset time period. The time information is the current time information. Whether the current time is within a preset time period is judged to judge whether to calibrate the blood pressure detection apparatus.

Specifically, if the first information is user input information, the user input information is configured to indicate whether the user needs to calibrate the blood pressure detection apparatus. The blood pressure detection apparatus is calibrated according to the user's needs.

Optionally, in the embodiment of the present application, the blood pressure of the user can be continuously detected with a preset time period as a cycle. Each time the blood pressure of the user is detected, whether to perform calibration can be determined flexibly according to the first information, for example, the current time information and/or user input information, etc., that is, determining whether to obtain new blood pressure calibration information. On one hand, during a calibration period of blood pressure detection, the calibration information can be updated according to user's needs or periodically to improve the accuracy of blood pressure detection. On the other hand, it can also take into account the user experience. During a non-calibration period of blood pressure detection, long-term continuous blood pressure detection is performed based on the previous calibration information and the PPG signal obtained by continuous detection, and long-term blood pressure detection services are provided.

The blood pressure detection method provided by the present application are described above with reference to FIG. 5 to FIG. 12. The blood pressure detection apparatus provided by the present application is described below with reference to FIG. 13 to FIG. 19. It can be understood that the blood pressure detection apparatus described in the following embodiment may be an apparatus configured to perform the above blood pressure detection method. Related technical features can be referred to the related description above.

As shown in FIG. 13, a blood pressure detection apparatus 101 includes: a processor 11, the processor 11 configured to:
acquire blood pressure calibration information, the blood pressure calibration information being information obtained by processing a first pressure acting on a user and a first photo plethysmography (PPG) signal when the first pressure acts on the user;
acquire a second photo plethysmography (PPG) signal, and process the second PPG signal to obtain the user's initial blood pressure;
calibrate the initial blood pressure according to the blood pressure calibration information, and use the blood pressure obtained by calibration as the blood pressure of the user.

Optionally, in some other implementation manners, the processor 11 is configured to:
control a prompt module 15 to output a prompt signal, the prompt signal configured to prompt the user to apply a pressure to the blood pressure detection apparatus to form the first pressure;
control a first pulse wave detection module 13 to detect the first PPG signal when the first pressure acts on the user;
control a pressure detection module 14 to detect the first pressure;
receive the first pressure and the first PPG signal, and determine the blood pressure calibration information according to the first pressure and the first PPG signal.

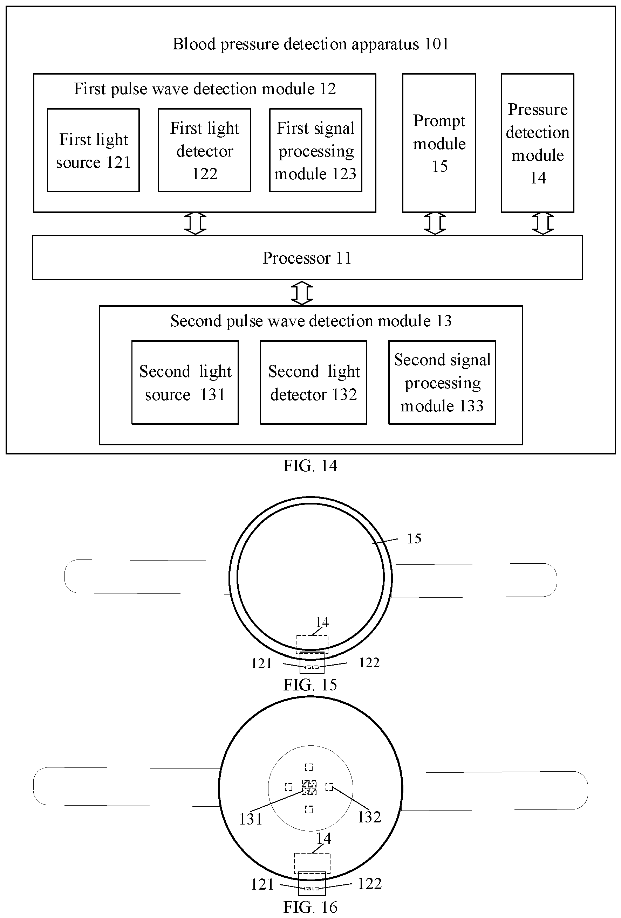

Optionally, as shown in FIG. 14, the blood pressure detection apparatus 101 in the embodiment of the present application may further include the above prompt module 15, the first pulse wave detection module 13, and the pressure detection module 14.

Optionally, the first pulse wave detection module 13 may include: a first light source 121, a first light detector 122 and a first signal processing module 123.

Optionally, the first light source 121 is configured to emit a first light signal to the first part of the user, and there is a first pressure acting on the first part of the user. The first light signal is reflected and/or transmitted by a blood vessel in the first part, and received by the first light detector 122. After the first light detector 112 performs photoelectric conversion on the received light signal, the formed electrical signal is transmitted to the first signal processing module 123 for signal processing to form the first PPG signal.

Optionally, the first light source 121 includes, but is not limited to, one or more point light sources, for example, a light-emitting diode (LED), a laser diode (LD), or an infrared emitting diode. It may further be a linear light source or a surface light source, which is not specifically limited in the embodiment of the present application. The first light source 121 may be configured to emit a first light signal of one or more target wavebands. As an example, the target waveband may be a red light waveband or a green light waveband.

Optionally, the first light detector 122 includes, but is not limited to, a photodiode (PD), a phototransistor, etc., which is configured to perform photoelectric conversion. The first signal processing module 123 may include: a signal processing circuit such as an amplifier circuit, a low-pass filter circuit, an analog-to-digital conversion circuit, etc., for optimizing a signal quality to improve the effect of blood pressure detection.

Optionally, the pressure detection module 14 may include a pressure sensor for sensing the first pressure. The pressure sensor includes, but is not limited to, a piezoelectric pressure sensor, a piezoresistive pressure sensor, a capacitive pressure sensor, and an inductive pressure sensor, or other types of pressure sensors, which is not specifically limited in the embodiment of the present application.

Specifically, the first light source 121 and the first light detector 122 may be located on the same side or an opposite side of the first part of the user, and are configured to receive a reflected light and/or transmitted light after passing through the first part to form the first PPG signal.

As an example, the first light source 121 and the first light detector 122 are both located on the same side of the first part of the user. For example, the blood pressure detection apparatus 101 is arranged on a smart watch. The first light source 121 and the first light detector 122 are arranged adjacently, which can be located on a side of the smart watch or in a button on the side of the smart watch.

Optionally, the pressure detection module 14 can be arranged on the side of the smart watch together with the first light source 121 and the first light detector 122, which can be stacked and arranged with the first light source 121 and the first light detector 122, or alternatively, can also be arranged horizontally with the first light source 121 and the first light detector 122.

Specifically, the processor 11 is configured to control a prompt module 12 to output a prompt signal, the prompt signal configured to prompt the user to apply a pressure to the blood pressure detection apparatus to form the first pressure.

Optionally, the prompt signal includes, but is not limited to, one or more of a text signal, an image signal, a sound signal, a vibration signal, or a light signal, and is intended to be configured to interact with the user. Correspondingly, the prompt module 12 includes, but is not limited to, a functional unit such as a display unit, a light-emitting unit, a sound unit, or a vibration unit.

After receiving the prompt signal, the user applies a pressure to the blood pressure detection apparatus 101 by the first part to generate the first pressure at the first part. As an example, the prompt module 12 is a display unit. For example, a display screen of a smart watch can be multiplexed as the prompt module 12. The display screen is configured to output a prompt signal, such as outputting a picture or video of a pressing gesture shown in FIG. 7. After the user receives the prompt signal, the finger is pressured on a side of the watch in the method shown in FIG. 7, and the finger is pressured on a button of the side of the watch, that is, pressed on a position corresponding to the first light source 121 and the first light detector 122.

Optionally, the prompt signal may be configured to prompt the user to change an intensity of pressure applied to the blood pressure detection apparatus 101 from large to small or from small to large, and then the first pressure detected by the pressure detection module 14 changes from large to small or from small to large. The user applies a pressure to the blood pressure detection apparatus 101 in this way. A change trend of the first pressure is the same. The processor 11 can conveniently sort the first PPG signals corresponding to the first pressure according to the magnitude of the first pressure, to obtain the envelope signal with a good signal quality, for detecting the blood pressure of the user, to obtain the blood pressure correction information.

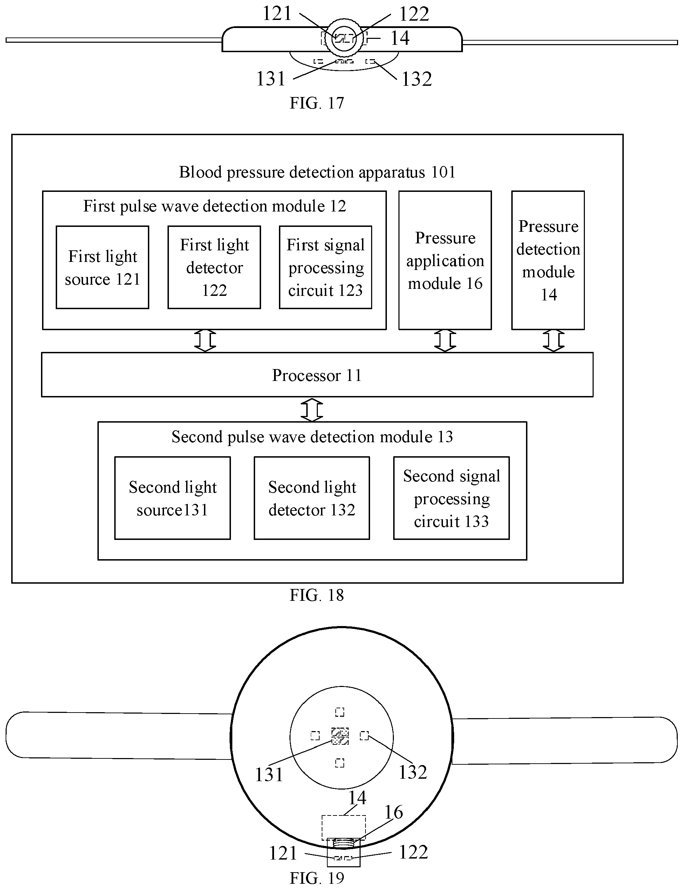

FIG. 15 to FIG. 17 show a top view, a bottom view and a side view of a smart watch. Specifically, FIG. 15 is a top view of the smart watch, that is, the front of the smart watch; FIG. 16 is a bottom view of the smart watch, that is, the back of the smart watch; and FIG. 17 is the side view of the smart watch, that is, a side of the smart watch.

As shown in FIG. 15 to FIG. 17, the first light source 121 and the first light detector 122 are arranged on a side button of the smart watch, and the pressure detection module 14 is arranged on a side of the first light source 121 and the first light detector 122 close to the inside of the watch, and is stacked and arranged with the first light source 121 and the first light detector 122.

It should be described that in the embodiments shown in FIG. 15 to FIG. 17, the pressure detection module 14 may be located inside the watch, and the first light source 121 and the first light detector 122 may be arranged inside the button. Therefore, in FIG. 15 to FIG. 17, the pressure detection module 14, the first light source 121, and the first light detector 122 are identified by adopting a dashed frame.

Optionally, the processor 11 is further configured to: control a second pulse wave detection module 13 to detect the second PPG signal of the user;
receive the second PPG signal, and process the second PPG signal by applying a pulse wave analysis method or a pulse wave transit time measurement method, to obtain the user's initial blood pressure.

Optionally, as shown in FIG. 14, the blood pressure detection apparatus 101 of the embodiment of the present application may further include the above second pulse wave detection module 13.

As an example, the second pulse wave detection module 13 may include: a second light source 131, a second light detector 132, and a second signal processing module 133.

Specifically, the second light source 131 may be a point light source, the number of which is one or more, configured to transmit second light signals of different target wavebands to the second part of the user. After the second light signals passing through a blood vessel of the second part of the user, it is received by the second light detector 132. The number of the second light detector 132 can also be one or more. After the one or more second light detectors 132 perform photoelectric conversion on the light received, the formed electrical signal is transmitted to the second signal processing module 133 for signal processing to form a second PPG signal.

Optionally, the second light source 131 and the second light detector 132 may both be located on the same side of the second part of the user. For example, the blood pressure detection apparatus 101 is arranged on a smart watch. The second light source 131 and the second light detector 132 are arranged adjacently, which can be located on the back of the smart watch.

As shown in FIG. 15 to FIG. 17, the smart watch includes a plurality of second light sources 131 and a plurality of second light detectors 132, where the plurality of second light sources 131 can emit second light signals of at least two different wavebands. The plurality of second light sources 131 are arranged at the center part of the back of the watch, and the plurality of second light detectors 132 surround the plurality of second light sources 131.

As shown in FIG. 14, in the embodiment of the present application, the processor 11 is configured to perform signal interaction with the first pulse wave detection module 12, the second pulse wave detection module 13, the prompt module 15, and the pressure detection module 14, which may be configured to generate a control signal to control the first pulse wave detection module 12, the second pulse wave detection module 13, the prompt module 15 and the pressure detection module 14 to perform a corresponding functional action. At the same time, it can also be configured to receive the above first pulse wave detection module 12, the second pulse wave detection module 13 and the pressure detection module 14 to detect and obtain various signals, such as the first pressure, the first PPG signal, and the second PPG signal, etc., for signal processing the various signals, so as to obtain the blood pressure detection result.

Optionally, in some other implementation manners, the processor 11 is configured to:
drive a pressure application module 16 to apply the first pressure to the user to form the first pressure;
control a first pulse wave detection module 12 to detect the first PPG signal when the first pressure acts on the user;
receive the first pressure and the first PPG signal, and determine the blood pressure calibration information according to the first pressure and the first PPG signal.

Optionally, in some implementation manners, the first pressure received by the processor 11 may be the first pressure sent to it by the pressure application module 16.

Optionally, in some other implementation manners, the processor 11 is further configured to: control the pressure detection module 14 to detect the first pressure. The first pressure received by the processor 11 is the first pressure sent to it by the pressure detection module 14.

Optionally, as shown in FIG. 18, the blood pressure detection apparatus 101 in the embodiment of the present application may further include the above pressure application module 16 and the first pulse wave detection module 12.

Optionally, in some implementation manners, the blood pressure detection apparatus 101 may further include: the pressure detection module 14.

Further, the blood pressure detection apparatus 101 may further include: a second pulse wave detection module 13.

Specifically, in the embodiment of the present application, the first pulse wave detection module 12, the second pulse wave detection module 13 and the pressure detection module 14 may be referred to the related description of FIG. 14 above, which will not be described in detail here.

In the embodiment of the present application, the processor 11 is configured to control the pressure application module 16 to apply a pressure to the user to form a first pressure to act on. Optionally, the processor 11 is configured to control the pressure application module to change an intensity of pressure applied to the user from large to small or from small to large. Therefore, the formed first pressure changes from large to small or from small to large. In some implementation manners, the pressure application module 16 includes, but is not limited to, a motor drive module or other types of drive modules, which is not specifically limited in the embodiment of the present application.

Optionally, if the first light source 121 and the first light detector 122 are arranged on a button of the watch, the pressure application module 16 may be arranged on one side of the button to provide a pressure to the button.

FIG. 19 shows a bottom view of a smart watch including a pressure application module 16, that is, a schematic diagram of a back of the smart watch.

As an example, as shown in FIG. 19, the pressure application module 16 may be arranged between the first light source 121, the first light detector 122, and the pressure detection module 14, so that the first light source 121 and the first light detector 122 perform detection of the first PPG signal, which also facilitates it to generate a first pressure by applying a pressure to a module where the first light source 121 and the first light detector are located through it, and further applying a pressure to the user. In addition, the pressure application module 16 and the pressure detection module 14 are arranged adjacently, which also facilitates pressure detection by the pressure detection module 14.

It can be understood that, in addition to the pressure application module 16, other functional modules in FIG. 18 and FIG. 19 can be referred to the related descriptions of FIG. 15 to FIG. 18 above, which will not be described here. In addition, FIG. 15 to FIG. 17 and FIG. 19 only take a smart watch as an example to describe the related structure of the blood pressure detection apparatus 101 provided in the embodiment of the present application, and do not limit the blood pressure detection apparatus 101 in the embodiment of the present application to be only arranged on the smart watch. It can also be arranged in any other type of electronic device such as a mobile phone, a computer, or a biological information detection device, which is not specifically limited in the embodiment of the present application.

In addition, with respect to the processor 11, in addition to being configured to implement the related functions described in FIG. 13, FIG. 14 and FIG. 18 above, it may be further configured to realize the related steps in the blood pressure detection method 100 above.

With respect to the first pressure and the first PPG signal, the processor 11 is configured to: sort the first PPG signal according to a magnitude of the first pressure to form an envelope signal of the first PPG signal; determine the first blood pressure of the user according to a waveform parameter of the envelope signal and the preset function equation; and use the first blood pressure as the blood pressure calibration information.

Optionally, with respect to the calibration process of the blood pressure calibration information and the initial blood pressure, the processor 11 may be configured to: use the initial blood pressure as the alternating current component of the blood pressure of the user, use the blood pressure calibration information as the direct current component of the blood pressure of the user, and calibrate to obtain the blood pressure of the user.

In some implementation manners, the processor 11 is configured as before acquiring the second PPG signal, and the processor 11 is further configured to: determine if the user is in an exercise state;
if not, the processor 11 is configured to: acquire the second PPG signal;
if so, the processor 11 is configured to: re-determine whether the user is in the exercise state after an interval preset time period.

In some implementation manners, the processor 11 is configured to: determine if an accelerate value of the accelerometer is within the range of the preset threshold, and determine if the user is in an exercise state.

Optionally, in some implementation manners, the blood pressure detection apparatus 101 in the embodiment of the present application may include an accelerometer or other types of motion sensors. In some other implementation manners, the accelerometer or other types of motion sensors may also be arranged in the electronic device where the blood pressure detection apparatus 101 is located, which is not specifically limited in the embodiment of the present application.

Optionally, the processor 11 is configured to: acquire the first PPG signal at the first part of the user;
acquire the second PPG signal at a second part of the user, where the second part is different from the first part.

Optionally, the first part is a finger of the user and the second part is a wrist of the user.

An embodiment of the present application further provides a blood pressure detection apparatus, including a processor and a memory, the memory configured to store a computer program, and the processor configured to call the computer program to execute the blood pressure detection method 100 in any one of the above embodiments of the present application.

An embodiment of the present application further provides an electronic device, and the electronic device may include the blood pressure detection apparatus 101 in any one of the above embodiments of the present application.

Optionally, the electronic device may be a smart watch or a mobile phone, and the blood pressure detection apparatus 101 may be arranged on any surface of the electronic device, for example, may be arranged on the back or side of the electronic device.

Further, the above blood pressure detection apparatus 101 may be arranged in a button on the surface of the electronic device, and the button may be configured only to realize blood pressure detection, or may realize other functions of the electronic device on the basis of realizing blood pressure detection.

It should be understood that the processing unit or the processor of the embodiment of the present application may be an integrated circuit chip with signal processing capability. In an implementation process, steps of the foregoing method embodiments may be completed by using an integrated logical circuit of hardware in the processor or an instruction in a form of software. The processor may be a general purpose processor, a digital signal processor (DSP), an application-specific integrated circuit (ASIC), a field programmable gate array (FPGA) or another programmable logic device, a discrete gate or transistor logic device, or a discrete hardware component. The methods, the steps, and the logical block diagrams disclosed in the embodiments of the present application can be implemented or executed. It may be understood that the blood pressure detection apparatus in the embodiment of the present application may further include a memory unit or a memory, and the memory may be a volatile memory or a nonvolatile memory, or may include a volatile memory and a nonvolatile memory.

An embodiment of the present application further provides a computer readable storage medium, where the computer readable storage medium stores one or more programs, and the one or more programs include an instruction. When the instruction is executed by a portable electronic device that includes a plurality of application programs, the portable electronic device can execute the method in the embodiment shown above.

The embodiment of the present application also provides a computer program including instructions that, when executed by a computer, cause the computer to execute the method of the embodiment shown above.

Those of ordinary skill in the art may be aware that, units and algorithm steps of the examples described in the embodiments disclosed herein may be implemented by electronic hardware, or a combination of computer software and computer software. Whether these functions are performed by hardware or software depends on particular applications and designed constraint conditions of the technical solutions. Persons skilled in the art may use different methods to implement the described functions for every particular application, but it should not be considered that such implementation goes beyond the scope of the present application.

In the several embodiments provided in the present disclosure, it should be understood that the disclosed system, apparatus, and method may be implemented in another manner. For example, the described apparatus embodiment is merely an example. For example, the unit division is merely logical function division and may be other division in actual implementation. For example, a plurality of units or components may be combined or integrated into another system, or some features may be ignored or not performed. From another point of view, the displayed or discussed mutual coupling or direct coupling or communication connection may be indirect coupling or communication connection via some interfaces, devices or units, and may be in electrical, mechanical or other forms.

The units described as separate parts may be or may not be separated physically, and a component displayed as a unit may be or may not be a physical unit, namely, may be located in one place, or may be distributed on a plurality of network units. Some or all of the units may be selected to achieve the purposes of the solutions in the present embodiments according to actual needs.

In addition, functional units in the embodiments of the present disclosure may be integrated into one processing unit, or each of the units may exist alone physically, or two or more units are integrated into one unit.

When the functions are implemented in the form of a software functional unit and sold or used as an independent product, the functions may be stored in a computer-readable storage medium. Based on such an understanding, the technical solutions of the present application essentially, or the part contributing to the prior art, or some of the technical solutions may be implemented in a form of a software product. The software product is stored in a storage medium and includes several instructions for instructing a computer device (which may be a personal computer, a server, a network device, or the like) to perform all or some of the steps of the methods described in the embodiments of the present application. The foregoing storage medium includes: any medium that can store program code, such as a USB flash drive, a removable hard disk, a read-only memory (ROM), a random access memory (RAM), a magnetic disk, or an optical disc.

The foregoing descriptions are merely specific implementation manners of the present application, but the protection scope of the present application is not limited thereto, persons skilled in the art who are familiar with the art could readily think of variations or substitutions within the technical scope disclosed by the present application, and these variations or substitutions shall fall within the protection scope of the present application. Therefore, the protection scope of the present application shall be subject to the protection scope of the claims.

## Claims

1. A blood pressure detection method (100) applied to a blood pressure detection apparatus, the blood pressure detection apparatus comprising a processor that executes the blood pressure detection method (100), and the blood pressure detection method (100) comprising:
acquiring (S110) blood pressure calibration information, the blood pressure calibration information being information obtained by processing a first pressure acting on a user and a first photo plethysmography, PPG, signal when the first pressure acts on the user;
acquiring (S120) a second photo plethysmography signal when no pressure acts on the user, and processing the second photo plethysmography signal to obtain the user's initial blood pressure;
calibrating (S130) the initial blood pressure according to the blood pressure calibration information, and using the blood pressure obtained by calibration as the blood pressure of the user;
wherein the first pressure changes with time, the determining (S115) the blood pressure calibration information according to the first pressure and the first photo plethysmography signal, comprises:
sorting (S1 151) the first photo plethysmography signal corresponding to the first pressure according to a magnitude of the first pressure to form an envelope signal of the first photo plethysmography signal;
determining (S1152) the first blood pressure of the user according to the envelope signal;
using the first blood pressure as the blood pressure calibration information;
wherein calibrating (S130) the initial blood pressure according to the blood pressure calibration information, and using the blood pressure obtained by calibration as the blood pressure of the user, comprises:
using (S131) the blood pressure calibration information as a direct current component of the blood pressure of the user;
using the initial blood pressure as an alternating current component of the blood pressure of the user;
calibrating (S132) the alternating current component of the blood pressure of the user according to the direct current component of the blood pressure of the user, and using the blood pressure obtained by calibration as the blood pressure of the user.

2. The blood pressure detection method (100) according claim 1, wherein acquiring (110) blood pressure calibration information, comprises:
driving (S112) a pressure application module to apply a pressure to the user to form the first pressure;
controlling (S113) a first pulse wave detection module to detect the first photo plethysmography signal when the first pressure acts on the user;
controlling (S114) a pressure detection module to detect the first pressure;
receiving (S115) the first pressure and the first photo plethysmography signal, and determining the blood pressure calibration information according to the first pressure and the first photo plethysmography signal; and/or
acquiring (S110) blood pressure calibration information, comprises:
controlling (S 111) a prompt module to output a prompt signal, the prompt signal configured to prompt the user to apply a pressure to the blood pressure detection apparatus to form the first pressure;
controlling (S113) a first pulse wave detection module to detect the first photo plethysmography signal when the first pressure acts on the user;
controlling (S114) a pressure detection module to detect the first pressure;
receiving the first pressure and the first photo plethysmography signal, and determining (S115) the blood pressure calibration information according to the first pressure and the first photo plethysmography signal.

3. The blood pressure detection method (100) according claim 1 or 2, wherein acquiring (S120) a second photo plethysmography signal, and processing the second photo plethysmography signal to obtain the user's initial blood pressure, comprises:
controlling (S121) a second pulse wave detection module to detect the second photo plethysmography signal of the user;
receiving (S122) the second photo plethysmography signal, and processing the second photo plethysmography signal by applying a pulse wave analysis method or a pulse transit time measurement method, to obtain the initial blood pressure of the user.

4. The blood pressure detection method (100) according to any one of claims 1 to 3, wherein before acquiring (S120) a second photo plethysmography signal, the blood pressure detection method (100) further comprises:
determining (S140) whether the user is in an exercise state;
if not, acquiring the second photo plethysmography signal;
if so, re-determining whether the user is in the exercise state after an interval preset time period.

5. The blood pressure detection method (100) according to any one of claims 1 to 4, wherein acquiring the first photo plethysmography signal, comprises:
acquiring the first photo plethysmography signal at a first part of the user;
acquiring the second photo plethysmography signal, comprises:
acquiring the second photo plethysmography signal at a second part of the user, wherein the second part is different from the first part.

6. A blood pressure detection apparatus (101), comprising a processor (11), the processor (11) configured to:
acquire blood pressure calibration information, the blood pressure calibration information being information obtained by processing a first pressure acting on a user and a first photo plethysmography signal when the first pressure acts on the user;
acquire a second photo plethysmography signal when no pressure acts on the user, and process the second photo plethysmography signal to obtain the user's initial blood pressure;
calibrate the initial blood pressure according to the blood pressure calibration information, and use the blood pressure obtained by calibration as the blood pressure of the user;
wherein the first photo plethysmography signal changes with a magnitude of the first pressure;
the processor (11) is configured to: sort the first photo plethysmography signal according to a magnitude of the first pressure to form an envelope signal of the first photo plethysmography signal;
determine the first blood pressure of the user according to the envelope signal;
use the first blood pressure as the blood pressure calibration information;
the processor (11) is configured to:
use the blood pressure calibration information as a direct current component of the blood pressure of the user;
use the initial blood pressure as an alternating current component of the blood pressure of the user;
calibrate the alternating current component of the blood pressure of the user according to the direct current component of the blood pressure of the user, and use the blood pressure obtained by calibration as the blood pressure of the user.

7. The blood pressure detection apparatus (101) according to claim 6, wherein the processor (11) is configured to:
drive a pressure application module to apply the first pressure to the user to form the first pressure;
control a first pulse wave detection module to detect the first photo plethysmography signal when the first pressure acts on the user;
control a pressure detection module to detect the first pressure;
receive the first pressure and the first photo plethysmography signal, and determine the blood pressure calibration information according to the first pressure and the first photo plethysmography signal; and/or
the processor (11) is configured to:
control a prompt module to output a prompt signal, the prompt signal configured to prompt the user to apply a pressure to the blood pressure detection apparatus to form the first pressure;
control a first pulse wave detection module to detect the first photo plethysmography signal when the first pressure acts on the user;
control a pressure detection module to detect the first pressure;
receive the first pressure and the first photo plethysmography signal, and determine the blood pressure calibration information according to the first pressure and the first photo plethysmography signal.

8. The blood pressure detection apparatus (101) according to claim 6 or 7, wherein the blood pressure detection apparatus (101) further comprises: the pressure application module electrically connected to the processor (11) and the first pulse wave detection module electrically connected to the processor (11); and/or
the blood pressure detection apparatus (101) further comprises: the prompt module electrically connected to the processor (11) and the first pulse wave detection module electrically connected to the processor (11).

9. The blood pressure detection apparatus (101) according to any one of claims 6 to 8, wherein the processor (11) is further configured to:
control a second pulse wave detection module to detect the second photo plethysmography signal of the user;
receive the second photo plethysmography signal, and process the second photo plethysmography signal by applying a pulse wave analysis method or a pulse transit time measurement method, to obtain the initial blood pressure of the user;
the blood pressure detection apparatus (101) further comprises: the second pulse wave detection module electrically connected to the processor (11).

10. The blood pressure detection apparatus (101) according to any one of claims 6 to 9, wherein the processor (11) is further configured to:
acquire the first photo plethysmography signal at a first part of the user;
acquire the second photo plethysmography signal at a second part of the user, wherein the second part is different from the first part.

11. An electronic device, comprising:
a blood pressure detection apparatus (101) according to any one of claims 6 to 10.

## Patentansprüche

1. Ein Blutdruckerfassungsverfahren (100), das auf eine Blutdruckerfassungsvorrichtung angewendet wird, wobei die Blutdruckerfassungsvorrichtung einen Prozessor umfasst, der das Blutdruckerfassungsverfahren (100) ausführt, und das Blutdruckerfassungsverfahren (100) umfasst:
Erfassen (S110) von Blutdruckkalibrierungsinformationen, wobei die Blutdruckkalibrierungsinformationen Informationen sind, die durch Verarbeiten eines ersten Drucks, der auf einen Benutzer einwirkt, und eines ersten Fotoplethysmographiesignals, PPG, erhalten werden, wenn der erste Druck auf den Benutzer einwirkt;
Erfassen (S120) eines zweiten Fotoplethysmographiesignals, wenn kein Druck auf den Benutzer einwirkt, und Verarbeiten des zweiten Fotoplethysmographiesignals, um den Ausgangsblutdruck des Benutzers zu erhalten;
Kalibrieren (S130) des anfänglichen Blutdrucks gemäß den Blutdruckkalibrierungsinformationen und Verwenden des durch die Kalibrierung erhaltenen Blutdrucks als den Blutdruck des Benutzers;
wobei sich der erste Druck mit der Zeit ändert, das Bestimmen (S115) der Blutdruckkalibrierungsinformation gemäß dem ersten Druck und dem ersten Fotoplethysmographiesignal, umfasst:
Sortieren (S1151) des ersten Fotoplethysmographiesignals, das dem ersten Druck entspricht, gemäß einer Größe des ersten Drucks, um ein Hüllensignal des ersten Fotoplethysmographiesignals zu bilden;
Bestimmen(S1152) des ersten Blutdrucks des Benutzers entsprechend dem Hüllkurvensignal;
Verwenden des ersten Blutdrucks als Kalibrierungsinformation für den Blutdruck;
wobei das Kalibrieren (S130) des anfänglichen Blutdrucks gemäß der Blutdruckkalibrierungsinformation und das Verwenden des durch die Kalibrierung erhaltenen Blutdrucks als den Blutdruck des Benutzers umfasst:
Verwenden (S131) der Blutdruckkalibrierungsinformationen als Gleichstromkomponente des Blutdrucks des Benutzers;
Verwenden des Ausgangsblutdrucks als Wechselstromkomponente des Blutdrucks des Benutzers;
Kalibrieren (S132) der Wechselstromkomponente des Blutdrucks des Benutzers entsprechend der Gleichstromkomponente des Blutdrucks des Benutzers, und Verwenden
den durch die Kalibrierung ermittelten Blutdruck als den Blutdruck des Benutzers.

2. Das Blutdruckerfassungsverfahren (100) nach Anspruch 1, wobei die Erfassung (110) Blutdruck-Kalibrierungsinformationen, umfasst:
Ansteuern (S112) eines Druckanwendungsmoduls, um einen Druck auf den Benutzer auszuüben, um den ersten Druck zu erzeugen;
Steueren (S113) eines ersten Pulswellenerfassungsmoduls, um das erste Fotoplethysmographiesignal zu erfassen, wenn der erste Druck auf den Benutzer wirkt;
Steuern (S114) eines Druckerfassungsmoduls, um den ersten Druck zu erfassen;
Empfangen (S115) des ersten Drucks und des ersten Fotoplethysmographiesignals, und
Bestimmen der Blutdruckkalibrierungsinformationen entsprechend dem ersten Druck und dem ersten Fotoplethysmographiesignal; und/oder
Erfassen (S110) von Blutdruckkalibrierungsinformationen, umfassend:
Steuern (S111) eines Aufforderungsmoduls zur Ausgabe eines Aufforderungssignals, wobei das Aufforderungssignal so konfiguriert ist, dass es den Benutzer auffordert, einen Druck auf die Blutdruckerfassungsvorrichtung auszuüben, um den ersten Druck zu bilden;
Steuern (S113) eines ersten Pulswellenerfassungsmoduls, um das erste Fotoplethysmographiesignal zu erfassen, wenn der erste Druck auf den Benutzer wirkt;
Steuern (S114) eines Druckerkennungsmoduls, um den ersten Druck zu erkennen;
Empfangen des ersten Drucks und des ersten Fotoplethysmographiesignals, und Bestimmen (S115) der Blutdruckkalibrierungsinformation entsprechend dem ersten Druck und dem ersten Fotoplethysmographiesignal.

3. Das Blutdruckerfassungsverfahren (100) nach Anspruch 1 oder 2, wobei das Erfassen (S120) eines zweiten Fotoplethysmographiesignals und das Verarbeiten des zweiten Fotoplethysmographiesignals, um den anfänglichen Blutdruck des Benutzers zu erhalten, umfasst:
Steuern (S121) eines zweiten Pulswellenerfassungsmoduls zur Erfassung des zweiten Fotoplethysmographiesignals des Benutzers;
Empfangen (S122) des zweiten Fotoplethysmographiesignals und Verarbeiten des zweiten Fotoplethysmographiesignals durch Anwenden eines Pulswellenanalyseverfahrens oder eines Pulslaufzeitmessverfahrens, um den Ausgangsblutdruck des Benutzers zu erhalten.

4. Das Blutdruckerfassungsverfahren (100) nach einem der Ansprüche 1 bis 3, wobei das Blutdruckerfassungsverfahren (100) vor dem Erfassen (S120) eines zweiten Fotoplethysmographiesignals weiterhin umfasst:
Bestimmen (S140), ob sich der Benutzer in einem Übungszustand befindet; wenn nicht, Erfassen des zweiten Fotoplethysmographiesignals;
wenn nicht, Erfassen des zweiten Fotoplethysmographiesignals;
wenn ja, erneutes Bestimmen, ob der Benutzer sich in dem Übungszustand befindet, nach einem voreingestellten Intervall Zeitspanne.

5. Das Blutdruckerfassungsverfahren (100) nach einem der Ansprüche 1 bis 4, wobei das Erfassen des ersten Fotoplethysmographiesignals umfasst:
Erfassen des ersten Fotoplethysmographiesignals an einem ersten Teil des Benutzers; Erfassen des zweiten Fotoplethysmographiesignals, umfassend:
Erfassen des zweiten Fotoplethysmographiesignals an einem zweiten Teil des Benutzers, wobei sich der zweite Teil von dem ersten Teil unterscheidet.

6. Eine Blutdruckerfassungsvorrichtung (101), die einen Prozessor (11) umfasst, wobei der Prozessor (11) konfiguriert ist zum:
Erfassen von Blutdruckkalibrierungsinformationen, wobei die Blutdruckkalibrierungsinformationen Informationen sind, die durch Verarbeiten eines ersten Drucks, der auf einen Benutzer wirkt, und eines ersten Fotoplethysmographiesignals, wenn der erste Druck auf den Benutzer wirkt, erhalten werden;
Erfassen eines zweiten Fotoplethysmographiesignals, wenn kein Druck auf den Benutzer einwirkt, und das zweite Fotoplethysmographiesignal zu verarbeiten, um den Ausgangsblutdruck des Benutzers zu erhalten;
Kalibrieren des anfänglichen Blutdrucks gemäß den Blutdruckkalibrierungsinformationen und den durch das Kalibrieren erhaltenen Blutdruck als den Blutdruck des Benutzers verwenden;
wobei sich das erste Fotoplethysmographiesignal mit der Größe des ersten Drucks ändert;
der Prozessor (11) konfiguriert ist zum Sortieren des Fotoplethysmographiesignals entsprechend einer Größe des ersten Drucks, um ein Hüllensignal des ersten Fotoplethysmographiesignals zu bilden;
Bestimmen des ersten Blutdrucks des Benutzers entsprechend dem Hüllkurvensignal; Verwenden des ersten Blutdrucks als Blutdruckkalibrierungsinformation;
der Prozessor (11) so konfiguriert ist zum Verwenden der Blutdruckkalibrierungsinformationen als Gleichstromkomponente des Blutdrucks des Benutzers;
Verwenden des Ausgangsblutdrucks als Wechselstromkomponente des Blutdrucks des Benutzers;
Kalibrieren der Wechselstromkomponente des Blutdrucks des Benutzers entsprechend der Gleichstromkomponente des Blutdrucks des Benutzers und den durch das Kalibrieren erhaltenen Blutdruck als den Blutdruck des Benutzers zu verwenden.

7. Die Blutdruckerfassungsvorrichtung (101) nach Anspruch 6, wobei der Prozessor (11) konfiguriert ist zum
Ansteuern eines Druckanwendungsmoduls, um den ersten Druck auf den Benutzer auszuüben, um den ersten Druck zu erzeugen; Steuern eines ersten Pulswellenerfassungsmoduls, um das erste Fotoplethysmographiesignal zu erfassen, wenn der erste Druck auf den Benutzer wirkt;
Steuern eines Druckerfassungsmodul, um den ersten Druck zu erfassen;
Empfangen des ersten Drucks und das erste Fotoplethysmographiesignal, und Bestimmen der Blutdruckkalibrierungsinformation entsprechend dem ersten Druck und dem ersten Fotoplethysmographiesignal; und/oder
der Prozessor (11) konfiguriert ist zum Steuern eines Aufforderungsmodul, um ein Aufforderungssignal auszugeben, wobei das Aufforderungssignal so konfiguriert ist, dass es den Benutzer auffordert, einen Druck auf die Blutdruckerfassungsvorrichtung auszuüben, um den ersten Druck zu bilden;
Steuern eines ersten Pulswellenerfassungsmoduls, um das erste Fotoplethysmographiesignal zu erfassen, wenn der erste Druck auf den Benutzer wirkt;
Steuern eines Druckerfassungsmoduls, um den ersten Druck zu erfassen;
Empfangen des ersten Drucks und das erste Fotoplethysmographiesignal und Bestimmen der Blutdruckkalibrierungsinformation gemäß dem ersten Druck und dem ersten Fotoplethysmographiesignal.

8. Die Blutdruckerfassungsvorrichtung (101) nach Anspruch 6 oder 7, wobei die Blutdruckerfassungsvorrichtung (101) ferner umfasst: das Druckanwendungsmodul, das elektrisch mit dem Prozessor (11) verbunden ist, und das erste Pulswellenerfassungsmodul, das elektrisch mit dem Prozessor (11) verbunden ist; und/oder
die Blutdruckerfassungsvorrichtung (101) ferner umfasst: das Aufforderungsmodul, das elektrisch mit dem Prozessor (11) verbunden ist, und das erste Pulswellenerfassungsmodul, das elektrisch mit dem Prozessor (11) verbunden ist.

9. Die Blutdruckerfassungsvorrichtung (101) nach einem der Ansprüche 6 bis 8, wobei der Prozessor (11) ferner konfiguriert ist zum
Steuern ein zweiten Pulswellenerfassungsmoduls, um das zweite Fotoplethysmographiesignal des Benutzers zu erfassen; Empfangen des zweiten Fotoplethysmographiesignals und das zweite Fotoplethysmographiesignal durch Anwendung eines Pulswellenanalyseverfahrens oder eines Pulslaufzeitmessverfahrens verarbeiten, um den Ausgangsblutdruck des Benutzers zu erhalten;
die Blutdruckerfassungsvorrichtung (101) umfasst ferner: das zweite Pulswellenerfassungsmodul, das elektrisch mit dem Prozessor (11) verbunden ist.

10. Die Blutdruckerfassungsvorrichtung (101) nach einem der Ansprüche 6 bis 9, wobei der Prozessor (11) ferner dazu konfiguriert zum:
Erfassen des ersten Fotoplethysmographiesignals an einem ersten Teil des Benutzers;
Erfassen des zweiten Fotoplethysmographiesignals an einem zweiten Teil des Benutzers, wobei sich der zweite Teil von dem ersten Teil unterscheidet.

11. Ein elektronisches Gerät, das Folgendes umfasst: eine Blutdruckerfassungsvorrichtung (101) nach einem der Ansprüche 6 bis 10.

## Revendications

1. Procédé de détection de pression artérielle (100) appliqué à un appareil de détection de pression artérielle, l'appareil de détection de pression artérielle comprenant un processeur qui exécute le procédé de détection de pression artérielle (100) et le procédé de détection de pression artérielle (100) comprenant :
l'acquisition (S110) d'informations d'étalonnage de la pression artérielle, les informations d'étalonnage de la pression artérielle étant des informations obtenues en traitant une première pression agissant sur un utilisateur et un premier signal de photopléthymographie, PPG, lorsque la première pression agit sur l'utilisateur ;
l'acquisition (S120) d'un second signal de photopléthymographie lorsqu'aucune pression n'agit sur l'utilisateur et le traitement du second signal de photopléthymographie pour obtenir la pression artérielle initiale de l'utilisateur ;
l'étalonnage (S130) de la pression artérielle initiale en fonction des informations d'étalonnage de la pression artérielle et l'utilisation de la pression artérielle obtenue par étalonnage comme pression artérielle de l'utilisateur ;
dans lequel la première pression change avec le temps, la détermination (S115) des informations d'étalonnage de la pression artérielle en fonction de la première pression et du premier signal de photopléthymographie comprend :
le tri (S1151) du premier signal de photopléthymographie correspondant à la première pression en fonction d'une grandeur de la première pression pour former un signal à enveloppe du premier signal de photopléthymographie ;
la détermination (S1152) de la première pression artérielle de l'utilisateur en fonction du signal à enveloppe ;
l'utilisation de la première pression artérielle comme informations d'étalonnage de la pression artérielle ;
dans lequel l'étalonnage (S130) de la pression artérielle initiale en fonction des informations d'étalonnage de la pression artérielle et l'utilisation de la pression artérielle obtenue par étalonnage comme pression artérielle de l'utilisateur comprennent :
l'utilisation (S131) des informations d'étalonnage de la pression artérielle comme composante de courant continu de la pression artérielle de l'utilisateur ;
l'utilisation de la pression artérielle initiale comme composante de courant alternatif de la pression artérielle de l'utilisateur ;
l'étalonnage (S132) de la composante de courant alternatif de la pression artérielle de l'utilisateur en fonction de la composante de courant continu de la pression artérielle de l'utilisateur et l'utilisation de la pression artérielle obtenue par étalonnage comme pression artérielle de l'utilisateur.

2. Procédé de détection de pression artérielle (100) selon la revendication 1, dans lequel l'acquisition (110) d'informations d'étalonnage de la pression artérielle comprend :
le fonctionnement (S112) d'un module d'application de pression pour appliquer une pression à l'utilisateur pour former la première pression ;
la commande (S113) d'un premier module de détection d'onde de pouls pour détecter le premier signal de photopléthymographie lorsque la première pression agit sur l'utilisateur ;
la commande (S114) d'un module de détection de pression pour détecter la première pression ;
la réception (S115) de la première pression et du premier signal de photopléthymographie et la détermination des informations d'étalonnage de la pression artérielle en fonction de la première pression et du premier signal de photopléthymographie ; et/ou
l'acquisition (S110) d'informations d'étalonnage de la pression artérielle comprend :
la commande (S111) d'un module d'invite pour émettre un signal d'invite, le signal d'invite étant configuré pour inviter l'utilisateur à appliquer une pression à l'appareil de détection de pression artérielle pour former la première pression ;
la commande (S113) d'un premier module de détection d'onde de pouls pour détecter le premier signal de photopléthymographie lorsque la première pression agit sur l'utilisateur ;
la commande (S114) d'un module de détection de pression pour détecter la première pression ;
la réception de la première pression et du premier signal de photopléthymographie et la détermination (S115) des informations d'étalonnage de la pression artérielle en fonction de la première pression et du premier signal de photopléthymographie.

3. Procédé de détection de pression artérielle (100) selon la revendication 1 ou 2, dans lequel l'acquisition (S120) d'un second signal de photopléthymographie et le traitement du second signal de photopléthymographie pour obtenir la pression artérielle initiale de l'utilisateur comprennent :
la commande (S121) d'un second module de détection d'onde de pouls pour détecter le second signal de photopléthymographie de l'utilisateur ;
la réception (S122) du second signal de photopléthymographie et le traitement du second signal de photopléthymographie en appliquant un procédé d'analyse d'onde de pouls ou un procédé de mesure de temps de transit du pouls pour obtenir la pression artérielle initiale de l'utilisateur.

4. Procédé de détection de pression artérielle (100) selon l'une quelconque des revendications 1 à 3, dans lequel, avant l'acquisition (S120) d'un second signal de photopléthymographie, le procédé de détection de pression artérielle (100) comprend en outre :
la détermination (S140) pour savoir si l'utilisateur est dans un état d'exercice ;
si tel n'est pas le cas, l'acquisition du second signal de photopléthymographie ;
si tel est le cas, la redétermination pour savoir si l'utilisateur est dans l'état d'exercice après une période de temps prédéfinie d'intervalle.

5. Procédé de détection de pression artérielle (100) selon l'une quelconque des revendications 1 à 4, dans lequel l'acquisition du premier signal de photopléthymographie comprend :
l'acquisition du premier signal de photopléthymographie au niveau d'une première partie de l'utilisateur ;
l'acquisition du second signal de photopléthymographie comprend :
l'acquisition du second signal de photopléthymographie au niveau d'une seconde partie de l'utilisateur, dans lequel la seconde partie est différente de la première partie.

6. Appareil de détection de pression artérielle (101) comprenant un processeur (11), le processeur (11) étant configuré pour :
acquérir des informations d'étalonnage de la pression artérielle, les informations d'étalonnage de la pression artérielle étant des informations obtenues en traitant une première pression agissant sur un utilisateur et un premier signal de photopléthymographie lorsque la première pression agit sur l'utilisateur ;
acquérir un second signal de photopléthymographie lorsqu'aucune pression n'agit sur l'utilisateur et traiter le second signal de photopléthymographie pour obtenir la pression artérielle initiale de l'utilisateur ;
étalonner la pression artérielle initiale en fonction des informations d'étalonnage de la pression artérielle et utiliser la pression artérielle obtenue par étalonnage comme pression artérielle de l'utilisateur ;
dans lequel le premier signal de photopléthymographie change avec une grandeur de la première pression ;
le processeur (11) est configuré pour : trier le premier signal de photopléthymographie en fonction d'une grandeur de la première pression pour former un signal à enveloppe du premier signal de photopléthymographie ;
déterminer la première pression artérielle de l'utilisateur en fonction du signal à enveloppe ;
utiliser la première pression artérielle comme informations d'étalonnage de la pression artérielle ;
le processeur (11) est configuré pour :
utiliser les informations d'étalonnage de la pression artérielle comme composante de courant continu de la pression artérielle de l'utilisateur ;
utiliser la pression artérielle initiale comme composante de courant alternatif de la pression artérielle de l'utilisateur ;
étalonner la composante de courant alternatif de la pression artérielle de l'utilisateur en fonction de la composante de courant continu de la pression artérielle de l'utilisateur et utiliser la pression artérielle obtenue par étalonnage comme pression artérielle de l'utilisateur.

7. Appareil de détection de pression artérielle (101) selon la revendication 6, dans lequel le processeur (11) est configuré pour :
faire fonctionner un module d'application de pression pour appliquer la première pression à l'utilisateur pour former la première pression ;
commander un premier module de détection d'onde de pouls pour détecter le premier signal de photopléthymographie lorsque la première pression agit sur l'utilisateur ;
commander un module de détection de pression pour détecter la première pression ;
recevoir la première pression et le premier signal de photopléthymographie et déterminer les informations d'étalonnage de la pression artérielle en fonction de la première pression et du premier signal de photopléthymographie ; et/ou
le processeur (11) est configuré pour :
commander un module d'invite pour émettre un signal d'invite, le signal d'invite étant configuré pour inviter l'utilisateur à appliquer une pression à l'appareil de détection de pression artérielle pour former la première pression ;
commander un premier module de détection d'onde de pouls pour détecter le premier signal de photopléthymographie lorsque la première pression agit sur l'utilisateur ;
commander un module de détection de pression pour détecter la première pression ;
recevoir la première pression et le premier signal de photopléthymographie et déterminer les informations d'étalonnage de la pression artérielle en fonction de la première pression et du premier signal de photopléthymographie.

8. Appareil de détection de pression artérielle (101) selon la revendication 6 ou 7, dans lequel l'appareil de détection de pression artérielle (101) comprend en outre :
le module d'application de pression raccordé électriquement au processeur (11) et le premier module de détection d'onde de pouls raccordé électriquement au processeur (11) ; et/ou
l'appareil de détection de pression artérielle (101) comprend en outre : le module d'invite raccordé électriquement au processeur (11) et le premier module de détection d'onde de pouls raccordé électriquement au processeur (11).

9. Appareil de détection de pression artérielle (101) selon l'une quelconque des revendications 6 à 8, dans lequel le processeur (11) est en outre configuré pour :
commander un second module de détection d'onde de pouls pour détecter le second signal de photopléthymographie de l'utilisateur ;
recevoir le second signal de photopléthymographie et traiter le second signal de photopléthymographie en appliquant un procédé d'analyse d'onde de pouls ou un procédé de mesure de temps de transit du pouls pour obtenir la pression artérielle initiale de l'utilisateur ;
l'appareil de détection de pression artérielle (101) comprend en outre : le second module de détection d'onde de pouls raccordé électriquement au processeur (11).

10. Appareil de détection de pression artérielle (101) selon l'une quelconque des revendications 6 à 9, dans lequel le processeur (11) est en outre configuré pour :
acquérir le premier signal de photopléthymographie au niveau d'une première partie de l'utilisateur ;
acquérir le second signal de photopléthymographie au niveau d'une seconde partie de l'utilisateur, dans lequel la seconde partie est différente de la première partie.

11. Dispositif électronique comprenant :
un appareil de détection de pression artérielle (101) selon l'une quelconque des revendications 6 à 10.
